# EUROPEAN PATENT SPECIFICATION

(11) **EP 2 836 844 B1**
(45) Date of publication and mention of the grant of the patent: **16.08.2017**
(21) Application number: 13750374.4
(22) Date of filing: 12.04.2013
(51) Int. Cl.: G01N 33/68

(54) **SPECIFIC SALIVARY BIOMARKERS FOR RISK DETECTION, EARLY DIAGNOSIS, PROGNOSIS AND MONITORING OF ALZHEIMER'S AND PARKINSON'S DISEASES**
IN SPEICHEL VORLIEGENDE BIOMARKER SPEZIFISCH FÜR DIE RISIKOBEURTEILUNG , FRÜHDIAGNOSE, PROGNOSE UND DAS MONITORING VON MORBUS ALZHEIMER UND MORBUS PARKINSON
BIOMARQUEURS SALIVAIRES SPÉCIFIQUES POUR LA DÉTECTION DE RISQUES, LE DIAGNOSTIC PRÉCOCE, LE PRONOSTIC ET LA SURVEILLANCE DE LA MALADIE D'ALZHEIMER ET DE LA MALADIE DE PARKINSON

(30) Priority: 13.04.2012 IN 1138DE2012
(43) Date of publication of application: 18.02.2015
(60) Divisional application: 17178876.3
(73) Proprietor: Oasis Diagnostics Corporation, Vancouver, WA 98686 (US)
(72) Inventor: RAI, Balwant, Haryana 125077 (IN); KAUR, Jasdeep, Kapurthala Punjab 144601 (IN)
(74) Representative: Spencer, Michael David
(86) International application number: PCT/IB2013/001407
(87) International publication number: WO 2013/153461

(56) References cited:
- US-A1- 2007 042 437
- BERMEJO-PAREJA FELIX ET AL: "Saliva levels of Abeta1-42 as potential biomarker of Alzheimer's disease: a pilot study", BMC NEUROLOGY, BIOMED CENTRAL LTD., LONDON, GB, vol. 10, no. 1, 3 November 2010 (2010-11-03), page 108, XP021074798, ISSN: 1471-2377, DOI: 10.1186/1471-2377-10-108
- PROLO P ET AL: "P3-103 Amyloid-beta, cytokine profiles and natural killer cell activity: supposed markers of Alzheimer's disease", NEUROBIOLOGY OF AGING, TARRYTOWN, NY, US, vol. 25, 1 July 2004 (2004-07-01), page S384, XP027082744, ISSN: 0197-4580 [retrieved on 2004-07-01]
- GERMAN ET AL: "Serum biomarkers for Alzheimer's disease: Proteomic discovery", BIOMEDICINE AND PHARMACOTHERAPY, ELSEVIER, FR, vol. 61, no. 7, 15 August 2007 (2007-08-15), pages 383-389, XP022201242, ISSN: 0753-3322, DOI: 10.1016/J.BIOPHA.2007.05.009
- BOSTON PAUL F ET AL: "Developing a simple laboratory test for Alzheimer's disease: measuring acetylcholinesterase in saliva - a pilot study", INTERNATIONAL JOURNAL OF GERIATRIC PSYCHIATRY, vol. 23, no. 4, April 2008 (2008-04), pages 439-440, XP002715436, ISSN: 0885-6230
- SHI MIN ET AL: "Salivary Tau Species are Potential Biomarkers of Alzheimer's Disease", JOURNAL OF ALZHEIMER'S DISEASE, vol. 27, no. 2, 2011, pages 299-305, XP002715437,
- ALVAREZ ET AL: "Serum TNF-alpha levels are increased and correlate negatively with free IGF-I in Alzheimer disease", NEUROBIOLOGY OF AGING, TARRYTOWN, NY, US, vol. 28, no. 4, 23 February 2007 (2007-02-23), pages 533-536, XP005901141, ISSN: 0197-4580, DOI: 10.1016/J.NEUROBIOLAGING.2006.02.012
- Singhal R.K, Anand, S.: "Salivary -42, IGF-I, IGF-II, Alpha Amylase, IL-1, and TNF-alpha in Alzheimer's Disease: A Useful diagnostic tool", , 3 August 2013 (2013-08-03), XP002721580, Retrieved from the Internet: URL:https://web.archive.org/web/2014022813 1418/http://wwhttp://www.webmedcentral.com /wmcpdf/Article_WMC004358.pdf [retrieved on 2014-03-12]
- KIM CHANG-BEOM ET AL: "Antibody-based magnetic nanoparticle immunoassay for quantification of Alzheimer's disease pathogenic factor.", JOURNAL OF BIOMEDICAL OPTICS MAY 2014, vol. 19, no. 5, 051205, May 2014 (2014-05) , pages 1-7, XP002721581, ISSN: 1560-2281

## Description

### FIELD OF THE INVENTION

The field of the invention is the use of salivary biomarkers for the detection of risk, early diagnosis, prognosis and monitoring of Alzheimer's and Parkinson's diseases according to one of a number of technological approaches, including, but not limited to, enzyme-linked immunosorbent assay [ELISA], homogeneous immunoassays, point-of-care devices, multiplex assays, biosensor technologies, mass spectrometry and others.

### BACKGROUND

Alzheimer's disease (AD) is a progressive neurodegenerative disorder in which neuro-degeneration starts decades before clinical symptoms appear (DeKosky ST, Marek K: "Looking Backward to Move Forward: Early Detection of Neurodegenerative Disorders," Science (2003) 302 (5646): 830-834). AD is the most common cause of dementia in the elderly, accounting for 50-60% of all cases (Blennow K, de Leon MJ, Zetterberg H: "Alzheimer's Disease": Lancet (2006); 368 (9533): 387-403.). The lifetime risk for AD between the ages of 65 and 100 is 33% for men and 45% for women, with an annual increase of 1-2% in the 7th decade to almost 60% in the 10^{th} decade. AD is very common and thus it is a major public health problem (Ferri CP, Prince M, Brayne C, et al. "Global Prevalence of Dementia: A Delphi Consensus Study". Lancet (2005) 366 (9503): 2112-2117).

Alzheimer's Disease International (ADI) estimates that globally there are currently 30 million people living with dementia, with 4.6 million new cases reported annually (one new case every 7 seconds). Statistics indicate that the number of people afflicted will reach over 100 million by 2050 (Ferri CP, Prince M, Brayne C, Brodaty H, Fratiglioni L, Ganguli M et al, "Global Prevalence of Dementia: a Delphi Consensus Study", Lancet (2005) 17; 366: 2112-7). Demographic aging is proceeding rapidly in China, India and Latin America and it is estimated that the number of older people in developing countries will increase by 200% compared to a much smaller estimated increase of 68% in the developed countries in the 30 years up to 2020 (WHO report: Global Burden of Disease (GBD) 2010). Unfortunately, in the United States, Medicare and most private health insurance companies fail to cover long-term care needed by sufferers of this debilitating disease.

Alzheimer's disease (AD) is a neurodegenerative disease of the central nervous system associated with progressive memory loss eventually resulting in dementia. Two pathological characteristics are observed in AD patients at autopsy: extracellular plaques and intracellular tangles in the hippocampus, cerebral cortex, and other areas of the brain essential for cognitive function (see Wostyn P, Audenaert K, De Deyn PP; "Choroidal Proteins Involved in Cerebrospinal Fluid Production may be Potential Drug Targets for Alzheimer's Disease Therapy," Perspectives in Medicinal Chemistry; 5:11-7; (2011); Sun X, Bhadelia R, Liebson E, Bergethon P, Folstein M, Zhu JJ, Mwamburi DM, Patz S, Qiu WQ; "The Relationship Between Plasma Amyloid-β Peptides and the Medial Temporal Lobe in the Homebound Elderly", Int. J. Geriatric Psychiatry 26 (6): 593-601 (2011).

The symptoms of AD manifest slowly and the initial signs may only be mild forgetfulness. In this early stage, individuals have a tendency to forget recent events, activities, the names of familiar people or things and may not be able to solve simple mathematical problems. As the disease progresses into moderate stages of AD, symptoms are more easily detected and become serious enough to cause people with AD or their family members to seek medical help. Moderate stage symptoms of AD include the inability to perform simple tasks such as grooming, and problems in speech, understanding, reading, and writing. Severe stage AD patients may become anxious or aggressive, may wander away from home and ultimately will need total care. The only definitive diagnostic test for AD relies upon analysis of brain tissue available only at autopsy following the death of the patient. Recently, new techniques including "multi-modal" methods combining the use of imaging techniques (e.g. PET scan, CT scan or MRI, for instance) with the detection of various biomarkers in cerebrospinal fluid [CSF] have been introduced, but these methods are highly invasive, expensive and so far have not been shown to be reliable in terms of sensitivity and specificity to detect AD accurately.

A simple, inexpensive and non-invasive diagnostic test that can be performed while the patient is alive, does not rely on the use of cerebrospinal fluid, removal of a biopsy specimen, or use of expensive and unreliable imaging techniques, would be extremely valuable in identifying AD early, as well as providing useful prognostic information. In addition, if such a tool could be used to monitor progression of the disease for patients treated with one of a number of therapeutic interventions currently under development by a number of pharmaceutical companies including Pfizer, Genentech, Medivation, Eli Lilly, Aphios and others, the technology would have broad appeal. Moreover, the development of a non-invasive diagnostic test using saliva as the specimen of choice would expand opportunities for testing in hard to reach populations and facilitate diagnosis in physician's offices, satellite clinics, outpatient facilities and other non-traditional testing facilities and would reduce the overall costs of AD patient care and the healthcare system in general. Further, if such a technology had the capability to provide accurate diagnostic information in patients suffering from Parkinson's disease in addition to AD, the technology would have yet greater appeal. The technology described herein is hereby demonstrated to meet these criteria.

In spite of huge advances in genomics in the last two decades that have seen the formation of an enormous number of microarray databases, data analysis procedures and generated protocols, Alzheimer's disease proteomics is still in its infancy (Heese, K. et al. (2000); "Induction of Rat L-Phosphoserine Phosphatase by Amyloid-β (1-42) is Inhibited by Interleukin-11," Neuroscience Letters 288 (1): 37-40; Huberman, M. et al. (1994) "Correlation of Cytokine Secretion by Mononuclear Cells of Alzheimer Patients and Their Disease Stage" J. Neuroimmunology 52 (2): 147-152; Robakis, N.K. et al. (1991) "Expression of the Alzheimer's Amyloid Precursor in Brain Tissue and Effects of NGF and EGF on its Metabolism" Clinical Neuropharmacology 14 (Supplement 1): S15-S23; Xia, M. et al. (1998) "Immunohistochemical Study of the B-Chemokine Receptors CCR3 and CCR5 and Their Ligands in Normal and Alzheimer's Disease Brains" American. J. Pathology 153 (1): 31-37; Collins, T. et al. (1985) "Cultured Human Endothelial Cells Express Platelet-Derived Growth Factor B Chain: cDNA Cloning and Structural Analysis" Nature (1985); 316 (6030): 748-750; Docherty, A.J.P. et al. (1985): "Sequence of Human Tissue Inhibitor of Metalloproteinases and its Identity to Erythroid- Potentiating Activity", Nature 318 (6041): 66-69; Foster, D.C. et al. (1994) "Human Thrombopoietin: Gene Structure, cDNA Sequence, Expression, and Chromosomal Localization" Proceedings of the National Academy of Sciences U.S.A. 91 (26): 13023-13027; Gasson, J.C. et al. (1985) "Molecular Characterization and Expression of the Gene Encoding Human Erythroid-Potentiating Activity" Nature 315 (6022): 768-771; Gray, A. et al. (1983) "Nucleotide Sequence of Epidermal Growth Factor cDNA Predicts a 128,000-Molecular Weight Protein Precursor", Nature 303: 722-725; Gray, P.W. et al. (1984) "Cloning and Expression of cDNA for Human Lymphotoxin, a Lymphokine with Tumour Necrosis Activity", Nature 312 (5996): 721-724; Hohn, A. et al. (1990) "Identification and Characterization of a Novel Member of the Nerve Growth Factor / Brain-Derived Neurotrophic Factor Family", Nature 344 (6264): 339-341; Rosenthal, A. et al. (1991) "Primary Structure and Biological Activity of Human Brain-Derived Neurotrophic Factor", Endocrinology 129 (3): 1289-1294; Schall, T.J et al. (1990) "Molecular Cloning and Expression of a Receptor for Human Tumor Necrosis Factor", Cell 61 (2): 361-370; Schall, T.J. et al. (1988) "A Human T Cell-Specific Molecule is a Member of a New Gene Family", J. Immunology 141 (3): 1018-1025; Stetler-Stevenson, W.G. et al. (1990) "Tissue Inhibitor of Metalloproteinases-2 (TIMP-2) mRNA Expression in Tumor Cell Lines and Human Tumor Tissues", J. Biological Chemistry 265 (23): 13933-13938; Yoshimura, T. et al. (1993) "Cloning, Sequencing, and Expression of Human Macrophage Stimulating Protein (MSP, MST1) Confirms MSP as a Member of the Family of Kringle Proteins and Locates the MSP Gene on Chromosome 3", J. Biological Chemistry 268 (21): 15461-15468; Zsebo, K.M. et al. (1990) "Stem Cell Factor is Encoded at the S-Locus of the Mouse and is the Ligand for the c-kit Tyrosine Kinase Receptor", Cell 63 (1): 213-224; Burbach, G.J. et al. (2004) "Induction of Brain-Derived Neurotrophic Factor in Plaque-Associated Glial Cells of Aged APP23 Transgenic Mice", J. Neuroscience 24 (1Q): 2421-2430; Citron, M. et al. (1997) "Mutant Presenilins of Alzheimer's Disease Increase Production of 42-Residue Amyloid B-Protein in Both Transfected Cells and Transgenic Mice", Nat. Med. 3 (1): 67-72; Fahnestock, M. et al. (2002) "Neurotrophic Factors and Alzheimer's Disease: Are We Focusing on the Wrong Molecule?", J. Neural Transmission (62 Supplement): 241-252; Fiala, M. et al. (1998) "Amyloid-β Induces Chemokine Secretion and Monocyte Migration Across a Human Blood-Brain Barrier Model," Molecular Med. 4 (7): 480-489; Galasko, D. (2001) "Biological Markers and the Treatment of Alzheimer's Disease", Journal of Molecular Neuroscience (2001); 17(2):119-125; Higgins, G.A. et al. (2003) "Transgenic Mouse Models of Alzheimer's Disease: Phenotype and Application", Behavioral Pharmacology 14 (5-6): 419-438; Hohlfeld, R. et al. (2000) "The Neuroprotective Effect of Inflammation: Implications for the Therapy of Multiple Sclerosis", J. Neuroimmunology 107: 161-166; Li, X.-L. et al (2002) "Impairment of Long-term Potentiation and Spatial Memory in Leptin Receptor-Deficient Rodents," Neuroscience 113 (3): 607-615; Masliah, E. et al. (1995) "PDGF is Associated with Neuronal and Glial Alterations of Alzheimer's Disease", Neurobiol. Aging 16 (4): 549-556; Oddo, S. et al. (2003) "Triple-Transgenic Model of Alzheimer's Disease with Plaques and Tangles: Intracellular AB and Synaptic Dysfunction", Neuron 39 (3): 409-421; Power, D.A. et al. (2001) "Circulating Leptin Levels and Weight Loss in Alzheimer's Disease Patients", Dement. Geriatric Cognitive Disorders 12 (2): 167-170; Bertram, L. et al. (2007) "Systematic Meta-analyses of Alzheimer Disease Genetic Association Studies: The AlzGene Database", Nature Genetics 39 (1): 17-23 + 76 supplemental pages; Araujo, D.M. et al. (1994) "Induction of Immune System Mediators in the Hippocampal Formation in Alzheimer's and Parkinson's Diseases: Selective Effects on Specific Interleukins and Interleukin Receptors", Neuroscience 61 (4) 745-754; Baskin, F. et al. (1997) "Altered Apolipoprotein E Secretion in Cytokine Treated Human Astrocyte Cultures", J. Neurological Sciences 148 (1): 15-18; Birecree, E. et al. (1991) "Epidermal Growth Factor and Its Receptor in the Developing Human Nervous System", Dev. Brain Research 60 (2): 145-154; Cheng, B. et al. (1992) "Glucose Deprivation Elicits Neurofibillary Tangle-like Antigenic Changes in Hippocampal Neurons: Prevention by NGF and bFGF", Experimental Neurology 117 (2): 114-123; Gray, C.W. et al. (1993) "Induction of β-Amyloid Precursor Protein Isoform mRNAs by bFGF in Astrocytes", Neuroreport 4 (6): 811-814; Hashimoto, Y. et al. (2003) "The Cytoplasmic Domain of Alzheimer's Amyloid-B Protein Precursor Causes Sustained Apoptosis Signal- Regulating Kinase 1/c-Jun NH2-Terminal Kinase-Mediated Neurotoxic Signal via Dimerization", J. Pharmacology Exp. Ther. 306 (3): 889-902; Hays, S.J. (1998) "Therapeutic Approaches to the Treatment of Neuroinflammatory Diseases", Current Pharm. Des. 4 (4): 335-348; Huberman, M. et al. (1994) "Correlation of Cytokine Secretion by Mononuclear Cells of Alzheimer Patients and Their Disease Stage", J. Neuroimmunology 52: 147-152; Satoh, J. et al. (2000) "β-Catenin Expression in Human Neural Cell Lines Following Exposure to Cytokines and Growth Factors", Neuropathology 20 (2): 113-123; Satoh, J. et al. (2000) "Amyloid Precursor Protein B-Secretase (BACE) mRNA Expression in Human Neural Cell Lines Following Induction of Neuronal Differentiation and Exposure to Cytokines and Growth Factors", Neuropathology 20 (4): 289-296; Treanor, J.J.S. et al. (1991) "Low Affinity Nerve Growth Factor Receptor Binding in Normal and Alzheimer's Disease Basal Forebrain", Neuroscience Letters 121 (1-2): 73-76; Yankner, B.A. et al. (1990) "Nerve Growth Factor Potentiates the Neurotoxicity of B Amyloid", Proceedings of the National Academy of Sciences USA 87: 9020-9023; Kovacs, E. (2001) "Serum Levels of IL-12 and IL-16 in Cancer Patients. Relation to the Tumour Stage and Previous Therapy", Biomedicine & Pharmacotherapy 55 (2): 111-116; Mohan, S. et al. (1997) "Serum Insulin-Like Growth Factor Binding Protein (IGFBP)-4 and IGFBP-5 Levels in Aging and Age-Associated Diseases", Endocrine 7 (1): 87-91; Pasinetti, G.M. et al. (2001) "From cDNA Microarrays to High-Throughput Proteomics. Implications in the Search for Preventive Initiatives to Slow the Clinical Progression of Alzheimer's Disease Dementia", Restorative Neurology and Neuroscience 18 (2,3): 137-142; Sjögren, M. et al. (2003) "Advances in the Detection of Alzheimer's Disease-Use of Cerebrospinal Fluid Biomarkers," Clinica Chimica Acta 332 (1-2): 1-10; Tham, A. et al. (1993) "Insulin-Like Growth Factors and Insulin-Like Growth Factor Binding Proteins in Cerebrospinal Fluid and Serum of Patients with Dementia of the Alzheimer Type," Journal of Neural Transmission 5 (3): 165-176; Turner, R.S. (2003) "Biomarkers of Alzheimer's Disease and Mild Cognitive Impairment: Are We There Yet?" Experimental Neurology 183 (1): 7-10; Bimonte-Nelson, H.A. et al. (2003) "Testosterone, but not Nonaromatizable Dihydrotestosterone, Improves Working Memory and Alters Nerve Growth Factor Levels in Aged Male Rats", Experimental Neurology 181 (2) 301-312; Capsoni, S. et al. (2000) "Alzheimer-Like Neurodegeneration in Aged Anti-nerve Growth Factor Transgenic Mice", Proc. Natl. Acad. Sci. U.S.A. 97 (12): 6826-6831; Hartbauer, M. et al. (2001) "Antiapoptotic Effects of the Peptidergic Drug Cerebrolysin on Primary Cultures of Embryonic Chick Cortical Neurons", J. Neural Transmission 108 (4): 459-473; Hellweg, R. et al. (1994) "Neurotrophic Factors in Memory Disorders", Life Sciences 55 (25-26): 2165-2169; Intebi, A.D. et al. (2003) "Alzheimer's Disease Patients Display Gender Dimorphism in Circulating Anorectic Adipokines", NeuroImmunoModulation 10 (6): 351-358; Kim, S.H. et al. (2002) "Brain-Derived Neurotrophic Factor Can Act as a Pronecrotic Factor Through Transcriptional and Translational Activation of NADPH Oxidase", J. Cell Biology 159 (5): 821-31; Verdier, Y. et al. (2004) "Binding Sites of Amyloid B-Peptide in Cell Plasma Membrane and Implications for Alzheimer's Disease", Curr. Protein Pept. Sci. 5 (1): 19-31; Verdier, Y. et al. (2004) "Amyloid β-Peptide Interactions with Neuronal and Glial Cell Plasma Membrane: Binding Sites and Implications for Alzheimer's Disease", J. Pept. Sci. 10 (5): 229-248; Windisch, M. et al. (1998) "Neurotrophic Activities and Therapeutic Experience with a Brain Derived Peptide Preparation", J. Neural Transmission (Supplement 3: 289-298).

A number of U.S. patents and patent applications have been published relating to methods for the diagnosis of AD, including U.S. Patent Numbers 4,728,605, 5,874,312, 6,027,896, 6,114,133, 6,130,048, 6,210,895, 6,358,681, 6,451,547, 6,461,831, 6,465,195, 6,475,161, and 6,495,335, and U.S. Patent Application Serial Numbers 11/580,405, 11/148,595, and 10/993,813.

In addition, the following patents and applications offer background and prior art in this area: United States Patent 7,794,948 teaches protein-based biomarkers and biomarker combinations that are useful in qualifying Alzheimer's disease (AD) status in a patient. Certain biomarkers of the invention may also be suitable for deployment as radio-labeled ligands in non-invasive imaging techniques such as Positron Emission Tomography (PET).

United States Patent application 20080261226 teaches biomarkers and diagnostic methods supporting the subject biomarkers based on the discovery of specific genes that have a two-fold or greater difference in gene expression in the spinal cord of a pre-symptomatic mouse model for amyotrophic lateral sclerosis [ALS]. Such biomarkers and diagnostic methods are useful for early detection of neural cell injury and death in acute and degenerative disease. While this patent is useful for this specific application, the patent did not define specific biomarkers for AD and in addition involves invasive procedures, particularly neural cell analysis and does not use non-invasive saliva samples.

United States Patent Application 20090263829 teaches biomarkers for AD, a method for detecting AD, a method of monitoring AD, and a kit for quantifying biomarkers for AD; however this patent is based upon broad spectrum biomarkers, collected in invasive fashion, using cerebrospinal fluid specimens, which are also highly expensive to collect and analyze.

United States Patent 7,851,172 teaches a method for quantifying a neurodegenerative disorder in a patient that includes obtaining a fluid sample from the subject, measuring a protein biomarker complex in said fluid sample and correlating the measurement with mild cognitive impairment or Alzheimer's disease status. The biomarkers include those that comprise at least one of a transthyretin protein and / or a prostaglandin-H2 D-isomerase protein, and at least one second, different protein selected from a list comprising transthyretin, prostaglandin-H2 D-isomerase, beta-2-microglobulin, cystatin C, superoxide dismutase [Cu-Zn], plasma retinol-binding protein, phosphatidylethanolamine-binding protein, carbonic anhydrase 2, prostaglandin-H2 D- isomerase, and / or serotransferrin protein. Again this patent incorporates the use of invasive and broad spectrum biomarkers measured in cerebrospinal fluid [CSF] specimens, which is a very expensive and invasive procedure.

United States Patent Application 20090226897 teaches the use of protein-based biomarkers and biomarker combinations in cerebrospinal fluid [CSF] that are useful in qualifying Alzheimer's disease status in a patient. The biomarkers can be detected by SELDI mass spectrometry, an expensive and technically difficult technique that requires highly expensive instrumentation. The technique is neither simple nor non-invasive.

United States Patent Application 20100167937 teaches the identification and use of serum biomarkers for neurodegenerative disease, including Alzheimer's disease, and related diseases. More specifically, this invention relates to the identification of protein biomarkers useful for the screening, diagnosis, and differentiation of Alzheimer's disease from Parkinson's disease, other neurodegenerative diseases, and normal controls, and in the monitoring of Alzheimer's disease severity and disease mechanisms in patients. The use of biomarkers under this patent application teaches only the use of invasive specimens including serum, but not saliva.

United States Patent Application 20090061457 teaches the use of the Apolipoprotein E3 protein as a biomarker for neurodegenerative disease, including Parkinson's disease, and other related diseases. More specifically, this invention relates to the application of the Apolipoprotein E3 protein as a tool for screening, diagnosis, and differentiation of Parkinson's disease from Alzheimer's disease, other neurodegenerative diseases, and normal controls. In this instance again, only invasive specimens are suggested including serum.

United States Patent 7,598,049 teaches a collection of serum proteinaceous biomarkers ("AD biomarkers"), which can be measured in peripheral biological fluid samples as an aid in the diagnosis of neurodegenerative disorders, particularly Alzheimer's disease and mild cognitive impairment (MCI). The invention further provides methods for identifying candidate agents for the treatment of Alzheimer's disease by testing prospective agents for activity in modulating AD biomarker levels. The patent does however rely upon the collection of invasive specimens and does not teach the use of non-invasive [saliva] samples.

United States Patent 7,833,513 teaches AD diagnosis by determining the level or function of insulin, insulin-like growths factors, their receptors and / or their downstream signaling molecules. The invention further relates to methods for treatment of AD by administering an insulin agonist and insulin - like growth factor agonist. The invention additionally provides an animal model of AD and methods of screening for agents useful in the treatment, amelioration, or prevention of AD. In this instance, the patent is based only upon the detection of biomarkers from histopathological sections. This requires that an invasive biopsy specimen must first be collected from the patient and assessed by highly trained pathologists, prior to disease diagnosis.

United States Patent 7,718,394 teaches the application of encephalotoxin produced by activated mononuclear phagocytes, present in individuals having neurological disease including neurodegenerative and neuro-inflammatory diseases, such as Alzheimer's disease (AD), HIV-1-associated dementia (HAD), Creutzfeldt-Jakob disease, mild cognitive impairment, prion disease, minor cognitive / motor dysfunction, acute stroke, acute trauma, or neuro-AIDS. Biochemical detection of encephalotoxin according to the methods of the invention will allow diagnosis of neurological disease in early, pre-symptomatic stages, thereby allowing early intervention in disease progression as well as identification of subjects or populations at risk for developing neurodegenerative disease. The methods of the invention also provide a mechanism for monitoring progression and treatment of neurological disease. This patent again utilizes only costly and invasive cerebrospinal fluid [CSF] biomarker sampling.

In United States Patent 7,897,361 methods and compositions relating to Alzheimer's disease are provided. Specifically, proteins that are differentially expressed in the Alzheimer's disease state relative to their expression in the normal state are provided. Proteins associated with Alzheimer's disease are identified and described. Methods of diagnosis of Alzheimer's disease using the differentially expressed proteins are also provided. Further evaluation indicates that this patent also relies on the use of invasive serum/plasma biomarker detection.

Despite the huge advances in genomics mentioned earlier that have resulted in comprehensive microarray databases, data analysis procedures and protocols, AD proteomics still remains an immature field of research. Currently, there are no valid biomarkers identified in patient samples (e.g., cerebrospinal fluid, blood, urine, etc.) that can be used to specifically diagnose, stratify, or monitor the progression or regression of AD or other forms of dementia (e.g., Parkinson's disease, Huntington's disease, Creutzfeldt-Jakob disease (CJD), multiple-infarct dementia, etc.). In the past two decades, hundreds of novel AD biomarkers have been discovered, but the results of numerous research efforts have not yet changed clinical practice in a significant way, because the vast majority of discovered biomarkers have not been validated. Literature supports the fact that very few studies are available on salivary biomarkers for AD and PD and no specific salivary markers have been characterized or validated for AD and PD.

### SUMMARY AND ADVANTAGES

In this patent, the words: "evaluate", "determinate", "discriminate" and "establish" are used for diagnosis and these words are interchangeable. "Normal healthy" refers to a value of zero on the clinical dementia rating scale [CRS] established by McKhann et al. (McKhann G, Drachman D, Folstein M, Katzman R, Price D, Stadlan E. "Clinical Diagnosis of Alzheimer's Disease: Report of the NINCDS-ADRDA Work Group Under the Auspices of the Department of Health and Human Services Task Force on Alzheimer's Disease. Neurology.1984; 34: 939-944) as well having other biochemical data (salivary biomarkers and other data) from these subjects being considered as normal.

The inventors have discovered sets or groups of biochemical markers present in the saliva of individuals, which are altered in individuals with Alzheimer's disease (AD). Accordingly the present invention is directed to methods of test set out in Claims 1, 3, 4 and 5 and further advantageous features in Claim 2. These sets of biomarkers (AD diagnostic, prognostic, risk detection biomarkers) may be used to diagnose or aid in the diagnosis of AD, prognosis of AD, risk of AD and/or to measure progression of AD in confirmed AD patients. The invention provides methods for the diagnosis of AD or aiding in the diagnosis of AD, prognosis of AD and risk of AD in an individual by quantitatively measuring the amount of each of a series of individual AD diagnostic biomarkers in saliva samples. Furthermore, this invention describes a means for quantification of multiple biomarkers, which, when measured in combination are strong indicators for diagnosis of AD, AD prognosis, early detection of AD and risk of AD in the individual. In addition, this patent identifies optimized subsets of biomarkers that, when used in tandem, are highly sensitive and specific for AD. The method described herein is a non-invasive, pain-free assessment / classification of cardiac risk factors as indicators for neurological disease using saliva as a diagnostic fluid, which, when used in conjunction with immunological assay platform technologies, such as, for example the VerOFy® Rapid Saliva Testing system from Oasis Diagnostics® Corporation [US Patent Numbers 7,618,591 and 7,927,548] introduces the possibility of a clinic, physician's office or home-based test for Alzheimer's disease. Such a test may be based upon one of a series of available rapid testing technologies available commercially, similar in nature to the VerOFy® technology, and may, or may not need a hand-held reading device to read and quantify the levels of the various biomarkers in patient saliva specimens. Available detection technologies for point-of-care applications include among others, lateral flow immunochromatography, latex agglutination, biosensor technology using fluorescence, chemiluminescence, magnetic bead-based technologies and others as well as alternate newer technologies including nanotechnology, biosensors and lab-on-a-chip methodologies. Incorporation of the salivary biomarker technology described herein with the VerOFy® platform device, or other similar technologies, will enable immediate results to be obtained through diagnosis at the point-of-care in cost effective fashion without sophisticated equipment or instrumentation. Such an innovation will result in a significant reduction in health care costs associated with diagnosis of AD.

Methods described herein may also be coupled with other technologies performed in the laboratory to non-invasively diagnose AD. Such technologies might include Enzyme Linked ImmunoSorbent Assay [ELISA], homogeneous immunoassays, chemiluminescence, mass spectrometry and many others.

As part of this disclosure the inventors have also developed a method by which salivary AD biomarkers and others, without limitation, are assigned a Salivary AD Index, which may be used to describe the ability of the biomarker (or combination of biomarkers) to discriminate between healthy individuals and AD (as an example). The Salivary AD Index is a reflection of the sensitivity, specificity, and overall accuracy of the salivary biomarkers to detect disease. The present invention provides a method for diagnosis, prognosis, risk detection for Alzheimer's disease (AD), comprising comparison of a measured level of a number of different AD biomarkers in a saliva sample from an individual seeking a diagnosis for AD compared to a reference level for each biomarker, wherein the different AD biomarkers comprise: cTnI, myoglobin, MMP-9, MMP-8, MMP-2, sICAM-1, myeloperoxidase, IL-4, and / or IL-5; B-type natiuretic peptide [BNP], IL-1α, IL-13, IL-6, IL-8, IL-10, TNF-α, IFN-γ, cTnI, VEGF, insulin, GLP-1 (active), GLP-1 (total), TREM1, leukotriene E4, Akt1, Aβ-40, Aβ-42, Fas ligand, or PSA, G-CSF, MIP-1α, IL-22, , IL-21, IL-15, IL-7, GM-CSF, IL-2, IL-12IL-17α, IL-1β, MCP, IL-32 or RANTES, sVCAM-1, sICAM-1, apolipoproteins A1, D and E, ischemia-modified albumin (IMA), fibronectin, myeloperoxidase (MPO), s. alpha-amylase, aspartate aminotransferase, lactate dehydrogenase, tissue factor activity, MCP-1, sCD-40, insulin-like growth factor I (IGF-I) and IGF-II.

In some embodiments, the method comprises comparing the measured value to a reference value for each AD diagnostic biomarker measured and may also comprise calculating the number of fold differences [i.e. 2-fold, 3-fold, etc.] between the measured value and the reference value. In other embodiments, the method comprises comparing the fold difference for each AD biomarker measured with a minimum fold difference value. In some embodiments, the measured levels are normalized against values from normal healthy individuals. In certain embodiments, the reference levels are obtained from measured values of the different biomarkers from samples in the saliva of human individuals without AD. In some embodiments, the reference levels are obtained from measured values of the different biomarkers from samples in the saliva of human individuals with AD. In some embodiments, the method comprises comparing the measured level of saliva AD diagnostic biomarkers to two reference levels for each biomarker. In some embodiments, the two reference levels for each biomarker comprise: (a) a reference level obtained from measured values of different biomarkers from samples in saliva of human individuals without AD; and (b) a reference level obtained from measured values of biomarkers from samples in the saliva of human individuals with AD. In some embodiments, the group of individuals without AD is a control population selected from an age-matched population, a degenerative control population, a non-AD neurodegenerative control population, a healthy age-matched control population, or a mixed population thereof. This method is a non-invasive, pain-free assessment / classification of neurological disease [AD and / or PD] using saliva as a diagnostic fluid, which, when used in conjunction with a point of care device, introduces the possibility of a home-based dementia assessment test. Use of the technology may also be applied to a multitude of other technologies available for diagnosis under laboratory and field conditions, with and without instrumentation. Such methods include, without limitation ELISA, homogeneous immunoassays, mass spectrometry, latex agglutination, fluorescence polarization immunoassay [FPIA], chemiluminescence immunoassays and biosensor technology, among others.

In another embodiment, the statistically significant difference is measured in terms of a p-value, where the p-value ranges from 0 to 0.05, while in other embodiments, parameters for the statistically significant difference comprise one or more of: a correlation of greater than 90% (r = 0.9 to r = 0.99); a p-value of between 0 and 0.05; a fold change in levels of greater than 20%, and a "d" score (a measure of the decrease or increase in specific levels of biomarkers in AD patients). In this case a d score of greater than 1 is defined for biomarkers whose levels increase and a d score of less than 1 is applicable for biomarkers whose levels decrease. In certain embodiments, the group of individuals without AD is a control population selected from an age-matched population, a degenerative control population, a non-AD neurodegenerative control population, a healthy age-matched control population, or a mixed population thereof. In some embodiments, all group individuals are a minimum of 60 years of age and a maximum of 85 years of age. In another aspect of the invention, laboratory based tests are used to measure the values and/or reference levels. Provided herein are methods for obtaining comparative values for measured levels relative to reference levels in biological fluid samples, particularly saliva. In any of the above embodiments, the comparison of the measured value and the reference value includes calculating a fold difference between the measured value and the reference value. In some embodiments, the measured value is obtained by quantifying the level of various AD diagnostic biomarkers in available patient samples, while in other embodiments the measured value is obtained from data from collection of samples carried out at three independent clinics

In further aspects, the sample may be a bodily fluid including, but not limited to blood, gingival crevicular fluid, serum, plasma, urine, nasal swab, cerebrospinal fluid, pleural fluid, synovial fluid, peritoneal fluid, amniotic fluid, gastric fluid, lymph fluid, interstitial fluid, tissue homogenate, cell extracts, saliva, sputum, stool, physiological secretions, tears, mucus, sweat, milk, semen, seminal fluid, vaginal secretions, fluid from ulcers and other surface eruptions, blisters, and abscesses, and extracts of tissues including biopsies of normal, and suspect tissues or any other constituents of the body which may contain the target substrate of interest.

Additional advantages of the invention will be set forth in part in the description which follows, and in part will be obvious from the description, or may be learned by practice of the invention. The advantages of the invention may be realized and attained by means of the instrumentalities and combinations particularly pointed out in the appended claims. Further benefits and advantages of the embodiments of the invention will become apparent from consideration of the following detailed description given with reference to the accompanying drawings, which specify and show preferred embodiments of the present invention.

### DETAILED DESCRIPTION

Before beginning a detailed description of the subject invention, mention of the following is in order. When appropriate, like reference materials and characters are used to designate identical, corresponding, or similar components in differing figure drawings. The figure drawings associated with this disclosure typically are not drawn with dimensional accuracy to scale, i.e., such drawings have been drafted with a focus on clarity of viewing and understanding rather than dimensional accuracy.

In the interest of clarity, not all of the routine features of the implementations described herein are shown and described. It will, of course, be appreciated that in the development of any such actual implementation, numerous implementation-specific decisions must be made in order to achieve the developer's specific goals, such as compliance with application- and business-related constraints, and that these specific goals will vary from one implementation to another and from one developer to another. Moreover, it will be appreciated that such a development effort might be complex and time-consuming, but would nevertheless be a routine undertaking of engineering for those of ordinary skill in the art having the benefit of this disclosure.

Inflammation and injury responses are invariably associated with neuron degeneration in AD, Parkinson's disease (PD), frontotemporal dementia, cerebrovascular disease, multiple sclerosis, and neuropathies. The inventors assert that the monitoring of relative concentrations of many secreted markers measured simultaneously in saliva specimens is a more sensitive method for monitoring the progression of disease than the absolute concentration of any single biochemical marker. The inventors describe herein a composite or array of sets of salivary biomarkers Quantitatively measured simultaneously, such sets of biomarkers consisting of [by way of example] antibodies bound to a solid support or proteins bound to a solid support, for the detection of inflammation and injury response markers associated with AD. Further, the inventors have discovered sets of markers (collectively termed "AD biomarkers") useful for the diagnosis of AD, as aids in the diagnosis of AD and for monitoring AD in AD patients (e.g., tracking disease progression in AD patients, which may be useful for tracking the effect of medical or surgical therapy in AD patients). The AD biomarkers are present in specific levels and may be quantified in various biological fluids of individuals. This invention teaches that certain AD biomarkers are present in biological fluids including the saliva of individuals, allowing collection of samples by simple, non-invasive procedures that are pain-free, particularly compared to the standard lumbar puncture procedure which is commonly used today to collect cerebrospinal fluid and blood samples for subsequent AD evaluation.

### Definitions

The present disclosure includes methods and compositions for the identification of biomarkers associated with Alzheimer's disease (AD). Biomarkers identified according to the methods and compositions disclosed can be used in diagnosing, stratifying, or monitoring the progression or regression of AD. The biomarkers may be used as drug targets to develop new drugs and monitor different therapies for the treatment of AD.

"Alzheimer's patient" and "AD patient" each refers to an individual who has been diagnosed with AD [for example, characterized as "diagnosed AD" by a Mini-Mental State Examination [MMSE] score or post mortem autopsy or been given a probable diagnosis of Alzheimer's disease (AD)]. AD includes individuals with a probable diagnosis of mild AD, moderate AD, or severe AD. "Non-AD patient" refers to a "normal" individual or sample from a "normal" individual who has or would be assessed by a physician as not having AD or mild cognitive impairment (MCI). In various embodiments, a non-AD patient may have an MMSE score (referenced in Folstein et al., J. Psychiatric Research (1975): 12: 1289-198), or would achieve an MMSE score in the range of 27 or above, or could have been assessed using other mental examination methods. On average people with Alzheimer's disease who do not receive treatment lose 2 to 4 points each year on the MMSE scoring system. An "individual" is a mammal, more preferably a human being. Mammals include, but are not limited to, humans, primates, livestock, domestic animals, sporting animals, rodents and pets. An "individual with mild AD" or "mild AD" is an individual who has been diagnosed with AD (for example, post mortem autopsy) or has been given a diagnosis of probable AD and would have been assessed either using the MMSE scoring system, and scored between 20 and 26 or would achieve a score of 20-26 upon MMSE testing or may have been assessed by other available mental examination methods. An "individual with moderate AD" or "moderate AD" is an individual who has been diagnosed with AD (for example, post mortem autopsy) or has been given a diagnosis of probable AD and would have been assessed using the MMSE scoring system and scored 10-19 or would achieve a score of 10-19 upon MMSE testing or who may have been assessed by other available mental examination methods. An "individual with severe AD" or "severe AD" is an individual who has been diagnosed with AD (for example, post mortem autopsy) or has been given a diagnosis of probable AD and would have been assessed using the MMSE scoring system and scored below 10 or would achieve a score of below 10 upon MMSE testing or who may have been assessed by other available mental examination methods.

As used herein, methods for "aiding diagnosis" refers to methods that assist in making a clinical determination regarding the presence, or nature, of AD or MCI (mild cognitive impairment), and may or may not be conclusive with respect to a definitive diagnosis. Accordingly, for example, a method of aiding diagnosis of AD can comprise measuring the quantity of one or a multiplicity of AD biomarkers in a biological sample from an individual.

The term "stratifying" refers to sorting individuals into different classes or strata based on the characteristics of the form and nature of AD. For example, stratifying a population of individuals with Alzheimer's disease involves assigning the individuals on the basis of the severity of the disease (e.g., mild, moderate, severe, etc.).

As used herein, the term "treatment" refers to the alleviation, amelioration, and / or stabilization of symptoms, as well as a delay in the progression of symptoms of a particular disorder, through the use of some external drug, device or technology. For example, "treatment" of AD includes any one or more of: the elimination of one or more symptoms of AD, reduction in one or more symptoms of AD, stabilization of the symptoms of AD (e.g., failure to progress to more advanced stages of AD), delay in progression (e.g., worsening) of one or more symptoms of AD, and regression (e.g., reverting back to an earlier stage of AD).

As used herein, the term "predicting" refers to making a judgment that an individual has a significantly enhanced probability of developing AD.

The term "prognosis" includes the likely outcome or course of AD.

By "therapeutic effect," "therapeutic activity" or "therapeutic action" it is meant a desired pharmacological activity of the agent against soluble tau oligomer, tau-Aβ1-42 complex [which is abbreviated elsewhere to Aβ-42, and / or tau-Aβ1-40 complex [abbreviated elsewhere to Aβ-40] for the treatment of AD, mild AD, moderate AD, and / or severe AD. For example, a drug (e.g., NSAID, statin, etc.) can be administered to a patient with AD, mild AD, moderate AD, or severe AD and the level of extracellular cerebrospinal fluid [CSF] soluble tau oligomer, tau-Aβ1-42 complex, and / or tau-Aβ1-40 complex can be measured to determine if the treatment has the desired therapeutic effect of lowering extracellular CSF levels of soluble tau oligomer, tau-Aβ1-42 complex, and/or tau-Aβ1-40 complex, for instance. If there is a reduction in CSF levels, the drug is having the desired therapeutic effect and could lead to an alleviation of the symptoms of AD. If CSF levels are not reduced, then the dose of the drug can be increased, discontinued, or another agent may be added in order to bring about the desired therapeutic effect. A number of new promising therapies are in development that will add to the battery of new therapeutic tools for AD.

Fold Difference: As used herein, the phrase "fold difference" refers to a numerical representation of the magnitude difference between a measured value and a reference value for a salivary AD biomarker. Fold difference is calculated mathematically by division of the measured numerical value by the numerical reference value. For example, if a measured value for an AD biomarker is 180 IU/ml, and the reference value is 60 IU/ml, the fold difference is 3. Alternatively, if a measured value for an AD biomarker is 60 IU/ml, and the reference value is 30 IU/ml, the fold difference is 2.

Reference Value: As used herein, a "reference value" can be an absolute value, a relative value, a value that has an upper and / or lower limit, a range of values, an average value, a median value, a mean value, a shrunken centroid value, or a value as compared to a particular control or baseline value. It is to be understood that other statistical variables may be used in determining the reference value. A reference value can be based on an individual sample value, such as for example, a value obtained from a sample from the individual with AD, but at an earlier point in time, or a value obtained from a sample from an AD patient other than the individual being tested, or a "normal" individual, that is an individual not diagnosed with AD. The reference value can be based on a large number of samples, such as from AD patients or normal individuals or based on a pool of samples including or excluding the sample to be tested.

Salivary AD Index: Salivary AD Index is defined as an index of diagnostic biomarkers used to explain the ability (sensitivity and specificity) of the salivary biomarker (or combination of specific biomarkers) to discriminate between healthy individuals and AD. The Salivary AD Index is a reflection of the sensitivity, specificity, and overall accuracy of the salivary biomarkers to detect disease. As used herein, the "Salivary AD Index" may also be defined as the ability of a biomarker (or combination of biomarkers) to discriminate between healthy individuals and different types of AD. For example the combination of the salivary biomarkers MMP-8, amylase and MYO (myoglobin) easily discriminates between healthy individuals and different types of AD as compared to combinations of other biomarkers. So, salivary MMP-8, amylase and MYO are defined as a Salivary AD Index because levels of these biomarkers are significantly higher in AD patients compared to those in healthy individuals

### Methods for Identifying Biomarkers

The sets of biomarkers used in the methods described herein include the set: cTnI, myoglobin, MMP-9, MMP-8, MMP-2, sICAM-1, myeloperoxidase [MPO], IL-4, and / or IL-5; B-type natiuretic peptide [BNP], IL-1α, IL-11, IL-10, TNF-α, IFN-γ, VEGF, insulin, GLP-1 (active), GLP-1 (total), TREM1, Leukotriene E4, Akt1, Aβ-40, Aβ-42, Fas ligand, PSA, G-CSF, MIP-1α, IL-22, IL-8, IL-21, IL-15, IL-6, IL-7, GM-CSF, IL-2, IL-12, IL-17α, IL-1β, MCP, IL-32 or RANTES, apolipoproteins A1, D and E, ischemia-modified albumin (IMA), fibronectin, s. alpha-amylase, aspartate aminotransferase, lactate dehydrogenase, tissue factor activity, MCP-1, sVCAM-1, sCD-40, insulin-like growth factor I (IGF-I) and IGF-II.

Accordingly, described herein are methods for identifying one or more additional biomarkers useful for diagnosis, aiding in diagnosis, assessing risk, monitoring, and / or predicting AD.

In certain aspects of the invention, quantitative levels of groups of biomarkers are obtained from sets of salivary samples collected from one or more individuals. The samples are selected such that they can be segregated into one or more subsets on the basis of the diagnostic value of the various biomarkers for the detection of AD. The measured values from the samples are compared to each other in order to identify those biomarkers which differ significantly within the subsets. The identified biomarkers that vary significantly within the subsets possess the optimum characteristics for diagnostic / prognostic purposes and may then be used in methods as aids in the diagnosis, monitoring, and / or prediction of AD. The process of comparing the measured values may be carried out by any method known in the art. Methods include traditional laboratory methods including enzyme linked immunosorbent assay [ELISA], fluorescence polarization immunoassay [FPIA] and homogeneous immunoassays, point of care tests using conventional lateral flow immunochromatography [LFA], quantitative point of care tests using determination of chemiluminescence, fluorescence, and magnetic particles, as well as latex agglutination, biosensors, gel electrophoresis, mass spectrometry [MS], gas chromatograph-mass spectrometry [GC-MS], and nanotechnology based methods, by way of example only.

### Methods of Evaluating AD

Described herein are methods for evaluating AD and for diagnosing or aiding in the diagnosis of AD by quantifying levels of sets of AD diagnostic biomarkers in salivary samples from various individuals and comparing the measured levels to reference levels, wherein the sets of biomarkers comprise: cTnI, myoglobin, MMP-9, MMP-8, MMP-2, sICAM-1, myeloperoxidase [MPO], IL-4, and / or IL-5; B-type natiuretic peptide [BNP], IL-1α, IL-11, IL-10, TNF-α, IFN-y, VEGF, insulin, GLP-1 (active), GLP-1 (total), TREM1, Leukotriene E4, Akt1, Aβ-40, Aβ-42, Fas ligand, PSA, G-CSF, MIP-1α, IL-22, IL-8, IL-21, IL-15, IL-6, IL-7, GM-CSF, IL-2, IL-12, IL-17α, IL-1β, MCP, IL-32 or RANTES, apolipoproteins A1, D and E, ischemia-modified albumin (IMA), fibronectin, s. alpha-amylase, aspartate aminotransferase, lactate dehydrogenase, tissue factor activity, MCP-1, sVCAM-1, sCD-40, insulin-like growth factor I (IGF-I), and IGF-II, any set of which may optionally comprise biomarker panels consisting of one, two, three, or more additional biomarkers. These sets of biomarkers are useful for a number of purposes, for example, assessment of the risk of developing AD, assessing the severity of the disease, monitoring AD post-diagnosis and others. AD biomarker detection includes but is not limited to secreted proteins or metabolites present in human biological fluids (that is, a biological fluid sample), such as for example, a body fluid including blood, gingival crevicular fluid, serum, plasma, urine, nasal swab, cerebrospinal fluid, pleural fluid, synovial fluid, peritoneal fluid, amniotic fluid, gastric fluid, lymph fluid, interstitial fluid, tissue homogenate, cell extracts, saliva, sputum, stool, physiological secretions, tears, mucus, sweat, milk, semen, seminal fluid, vaginal secretions, fluid from ulcers and other surface eruptions, blisters, and abscesses, and extracts of tissues including biopsies of normal, and suspect tissues or any other constituents of the body which may contain the target molecule of interest. As described herein, assessment of results may be qualitative or quantitative depending upon the specific method of detection employed.

In one aspect, the present invention provides methods of aiding diagnosis of Alzheimer's disease [AD] and diagnosing AD, by measuring and quantifying levels of each AD biomarker in a set of AD biomarkers in a salivary sample collected from a peripheral biological fluid sample from an individual, and comparing the measured levels to established reference levels for each of the biomarkers. In some embodiments, the set of AD diagnostic biomarkers comprises: IGF-I, IGF-II, Aβ-40, Aβ-42, alpha amylase, IL-1 beta, and TNF-alpha. In other embodiments, the set of AD diagnostic biomarkers comprises: cTnI, myoglobin, MMP-9, MMP-8, MMP-2, sICAM-1, myeloperoxidase, IL-4, and / or IL-5; B-type natiuretic peptide, IL-1α, IL-11, IL-10, TNF-α, IFN-γ, VEGF and insulin. Optionally, these sets may further comprise additional biomarkers such as Fas ligand, or PSA, G-CSF, MIP-1α, IL-22, IL-21, IL-15, IL-7, GM-CSF, IL-2, IL-4, IL-1α, IL-12, IL-17α, MCP, IL-32 or RANTES, sVCAM-1, sICAM-1, apolipoproteins A1, D and E, ischemia-modified albumin (IMA) and fibronectin.

Results of the quantification of various biomarker levels in the saliva of AD patients, leads to the differentiation of a priority listing of biomarkers (clustered by the methods described herein) in decreased ranking order with the highest to lowest ranked biomarkers within each cluster ranked based on values that are shown to be significantly elevated or significantly decreased in AD patients compared to age-matched normal healthy controls and against other neurodegenerative diseases that are not AD, such as for example Parkinson's disease ["PD"] and PN (neurodegenerative diseases that are not AD) compared to all control samples. Generally, a significant increase or decrease in the level of a given biomarker compared to an appropriate control may be indicative of AD.

While many subsets provide good sensitivity and specificity for AD diagnosis, of the various biomarker subsets investigated, the subset comprising IGF-I, IGF-II, Aβ-40, Aβ-42, alpha amylase, IL-1 beta, and TNF-alpha was found to be the best subset for diagnosis of AD compared to other biomarker subsets.

In a further aspect, this invention provides methods for monitoring the progression of AD in a diagnosed AD patient. As an example the inventors have shown that levels of the single marker, Aβ-42 are increased in AD patients with a questionable AD diagnosis (MMSE = 27-30) and that quantitative levels of Aβ-42 are increased in AD patients with mild AD (MMSE=20-25); Furthermore that Aβ-42 levels increase further as the severity of AD intensifies

In various embodiments, the sensitivity achieved by the use of defined sets of AD biomarkers such as one biomarker (Aβ-42), two biomarkers (A6-40 and Aβ-42), three biomarkers (Aβ-40, A6-42 and IGF-II), four biomarkers (Aβ-40, Aβ-42 , IGF-I and IGF-II) and so on as part of a method for diagnosing or aiding in the diagnosis of AD is at least 70%, 78%, 89% and 98% according to whether one, two, three or four biomarkers, respectively, are used.

In various embodiments, the specificity achieved using the set of defined AD biomarkers such as a set containing a single biomarker (Aβ-42), two biomarkers (Aβ-40 and Aβ-42), three biomarkers (Aβ-40, Aβ-42 and IGF-II) , four biomarkers (Aβ-40, Aβ-42, IGF-I and IGF-II) and so on as part of a method for diagnosing or aiding in the diagnosis of AD is at least 75%, at least 78%, at least 89% and at least 95% specificity, respectively.

In these embodiments, the overall accuracy achieved by the use of a defined set of AD biomarkers such as a set containing a single biomarker (Aβ-42), two biomarkers (Aβ-40 and Aβ-42), three biomarkers (Aβ-40, Aβ-42 and IGF-II), four biomarkers (Aβ-40, Aβ-42, IGF-I and IGF-II) and so on as part of a method for diagnosing or aiding in the diagnosis of AD is at least 78%, at least 80%, at least 88% and at least 95% accuracy, respectively.

In some embodiments, the sensitivity and / or specificity performance characteristics are measured against specimens from patients with a clinical diagnosis of AD. In certain embodiments of the invention, levels of AD biomarkers such as the biomarker set comprising Aβ-40, Aβ-42 and IGF-II are obtained from an individual at more than one time point. In certain embodiments individual assessments are carried out on individuals without any indication of AD, suspected AD, or risk of AD.

### Measuring Levels of AD Salivary Biomarkers

There are a number of statistical methods used for identifying biomarkers which vary significantly between subsets of salivary biomarkers, one of which is the conventional "t test". Biomarkers identified as being collectively useful as an aid in the diagnosis, monitoring, and / or prediction of AD rely on a significant difference between the subsets of salivary samples tested. Levels of biomarkers are "significantly different" when there is a probability that particular biomarker levels are significantly lower or higher than measured base line values. Further details are presented in the studies below.

The following studies are provided to illustrate the invention, but are not intended to limit in any way the scope of the invention.

### Study 1: Determination of the Optimum Set of Salivary Biomarkers

In order to determine the optimum set of biomarkers a study was performed, which included three patient groups: 100 Alzheimer's disease (AD) patients; 56 elderly non-demented controls without neurological disease or cognitive impairment and 51 Parkinson's disease (PD) patients. All AD cases included in this study were diagnosed with dementia according to the Diagnostic and Statistical Manual of Mental Disorders (DSM)-IV criteria (American Psychiatric Association: DSM-IV: Diagnostic and Statistical Manual of Mental Disorders, Washington DC: American Psychiatric Association (1994) and NINCDS-ADRDA criteria (McKhann G, Drachman D, Folstein M, Katzman R, Price D, Stadlan EM: "Clinical Diagnosis of Alzheimer's Disease: Report of the NINCDS-ADRDA Working Group Under the Auspices of the Department of Health and Human Services Task Force on Alzheimer's Disease," Neurology (1984) 34: 939-944), with verified evidence of cognitive decline (neuropsychological test battery, clinical mental examination, etc.) as well as evidence of impairment in social or occupational function. The mini-mental state examination (MMSE) was used to assess cognitive function (Folstein MF, Folstein SE, McHugh PR: "Mini-mental State -A Practical Method for Grading the Cognitive State of Patients for the Clinician," J Psychiatric Research (1975) 12: 189-198). The mean MMSE score for the enrolled AD patients was 17. All cases had an extensive biochemical work up performed including measurement of levels of vitamin B12 / folate, thyroid hormones and neuro-imaging analysis (brain MRI and / or CT scan). Classification of AD as mild, moderate and severe was determined, and the diagnosis of vascular dementia was excluded in all cases, using the established DSM-III-R criteria (American Psychiatric Association, DSM-III-R, Diagnostic and Statistical Manual of Mental Disorders, APA, Washington DC, USA 1987). The control group comprised family members or caregivers of the AD patients, all of whom underwent clinical interviews with a senior neurologist who confirmed completely normal cognitive and functional performance in this group. No formal neuropsychological biochemical or neuro-imaging analysis was performed on this group. The Parkinson's disease [PD] group was formulated from patients who had been diagnosed using the normal criteria of "probable PD" (Calne DB, Snow BJ, Lee C: "Criteria for Diagnosing Parkinson's Disease," Ann Neurology (1992) 32:125-127; Gelb DJ, Oliver E, Gilman S: "Diagnostic Criteria for Parkinson's Disease", Arch Neurology (1999) 56: 33-39. In an attempt to evaluate salivary biomarkers that are altered in AD and PD patients and 19 non demented age-matched controls, biomarker levels in saliva were measured using ELISA kits from various manufacturers as described below. MMP-2 and MMP-9 levels were quantified using kits from R&D Systems, (Minneapolis, Minnesota), IL-18 using a kit from Medical & Biological Laboratories Co (Naka-ku, Nagoya, Japan), cTnI, and CD31/PCAM-1, sICAM-2, sICAM-3 (Life Diagnostic, West Chester, PA), sVCAM-1, BNP, RANTES (Diaclone, Besancon, Cedex, France); GM-CSF, IL-2, IL-4, IL-1α, IL-12, IL-17α, IL-1β, MCP, IL-32, IFN-γ and TNF-α (Luminex, USA); alpha amylase, aspartate aminotransferase, lactate dehygrogenase (Salimetrics, USA); MYO and MPO (Biodesign International, Saco, ME), MCP-1 (AbD Serotec, Oxford, UK); sCD40 (HyTest Ltd, Turku, Finland); Tissue Factor Activity (St. Charles, MO USA) and MMP-8 (Human Quantikine, USA); Aβ-40, Aβ-42 (Biosource International, Invitrogen), IGF-I and IGF-II RIA (Van Wyk and Underwood antibody). Data were analyzed as discussed in detail in the description part of this invention.

Results: The set of biomarkers comprising salivary IGF-I, IGF-II, Aβ-40, Aβ-42, alpha amylase, IL-1beta, and TNF-alpha was found to be the best biomarker set in comparison to other sets in discriminating AD patients from normal controls (Table 1).

**Table 1 - Salivary Biomarkers Level in Different Types of AD, PD and Normal Healthy Individuals**

| **Mean Value (Standard Deviation)** | | | | | |
|---|---|---|---|---|---|
| Salivary Biomarkers Measured | Control Samples (Normal Healthy Individuals) | Parkinson's Disease Patients | Alzheimer's Disease Patients | P Value (Control and Parkinson's Disease ) | P Value (Control and Alzheimer's Disease) |
| Aβ-40 (pg/ml) | 34.4 (4.56) | 23.6 (3.78) | 12.12 (1.34) | <0.0001 | <0.0001 |
| TNF-α (pg/ml) | 68.89 (23.78) | 79.89 (12.45) | 267.76 (33.45) | <0.0001 | <0.0001 |
| IL-1-β (pg/ml) | 48.56 (34.75) | 67.56 (24.56) | 189.76 (33.89) | <0.0001 | <0.0001 |
| Aβ-42 (pg/ml) | 4.08 (2.45) | 5.78 (2.34) | 10.34 (1.45) | <0.0001 | <0.0001 |
| IGF-I (ng/ml) | 2.33 (1.23) | 1.87 (0.97) | 1.45 (0.57) | <0.0001 | <0.0001 |
| IGF-II (ng/dL) | 3.45 (2.78) | 2.45 (1.78) | 1.04 (0.68) | <0.0001 | <0.0001 |
| Alpha-amylase (U/ml) | 20.2 (10.3) | 38.78 (10.8) | 69.13 (14.51) | <0.005 | <0.005 |
| cTnI(ng/ml) | 1.34 (0.98) | 1.78 (1.32) | 1.76 (1.33) | 0.897 | 0.578 |
| Myoglobin (ng/ml) | 0.97 (1.34) | 0.96 (0.89) | 0.94 (0.85) | 0.467 | 0.893 |
| MMP-9 (ng/ml) | 73.3 (67.8) | 72.1 (69.2) | 72.3 (67.9) | 0.367 | 0.582 |
| MMP-8(ng/ml) | 156.7 (145.7) | 150.5 (167.8) | 151 (168) | 0.473 | 0.643 |
| MMP-2(ng/ml) | 78.4 (156.6) | 89.7 (89.3) | 89.4 (89.4) | 0.523 | 0.678 |
| sICAM-1(ng/ml) | 0.78 (0.67) | 0.78 (0.71) | 0.77 (0.72) | 0.674 | 0.463 |
| Myeloperoxidase [MPO] (ng/ml) | 12.45 (13.56) | 15.67 (12.45) | 15.89 (14.67) | 0.813 | 0.789 |
| IL-4 (ng/ml) | 14.67 (34.56) | 14.89 (35.78) | 14.99 (36.89) | 0.904 | 0.983 |
| IL-5 (ng/ml) | 23.09 (13.67) | 22.23 (23.81) | 24.24 (24.89) | 0.634 | 0.544 |
| B-type natiuretic peptide [BNP] (ng/ml) | 34.78 (12.67) | 32.89 (17.23) | 32.88 (19.56) | 0.453 | 0.643 |
| IL-1α (ng/ml) | 45.78 (45.78) | 43.89 (46.89) | 44.82 (45.09) | 0.612 | 0.786 |
| IL-11 (ng/ml) | 78.09 (78.02) | 77.23 (70.34) | 78.13 (73.65) | 0.892 | 0.632 |
| IL-10 (ng/ml) | 123.5 (67.03) | 127.5 (69.13) | 128.4 (68.24) | 0.922 | 0.653 |
| IFN-γ (ng/ml) | 67.89 (34.78) | 68.01 (35.98) | 68.34 (34.78) | 0.632 | 0.603 |
| VEGF (ng/ml) | 0.89 (1.23) | 0.89 (0.87) | 0.88 (0.86) | 0.612 | 0.813 |
| Insulin (ng/ml) | 0.08 (0.02) | 0.07 (0.03) | 0.05 (0.02) | 0.678 | 0.943 |
| GLP-1 (active) (ng/ml) | 1.34 (0.89) | 1.24 (0.98) | 1.26 (1.34) | 0.683 | 0.309 |
| GLP-1 (total) (ng/ml) | 19.23 (12.03) | 18.34 (11.22) | 19.24 (13.67) | 0.098 | 0.134 |
| TREM1 (ng/ml) | 13.67 (12.56) | 13.89 (13.65) | 13.87 (14.78) | 0.456 | 0.894 |
| Leukotriene E4 (ng/ml) | 13.98 (13.78) | 14.76 (14.86) | 14.89 (15.09) | 0.782 | 0.356 |
| Akt1 (ng/ml) | 0.83(1.33) | 0.84 (1.23) | 0.85 (1.03) | 0.233 | 0.623 |
| Fas ligand (pg/ml) | 1.34 (1.45) | 1.33 (1.34) | 1.23 (1.34) | 0.932 | 0.893 |
| PSA (ng/ml) | 1.34 (1.45) | 1.31 (1.23) | 1.32 (1.24) | 0.785 | 0.348 |
| G-CSF (ng/ml) | 2.45 (1.67) | 2.89 (1.56) | 2.91 (1.63) | 0.783 | 0.563 |
| MIP-1α (ng/ml) | 23.67 (12.56) | 23.98 (13.67) | 24.08 (14.89) | 0.924 | 0.521 |
| IL-22 (ng/ml) | 34.09 (34.09) | 35.12 (33.12) | 35.09 (34.12) | 0.783 | 0.532 |
| IL-8 (ng/ml) | 123.22 (34.65) | 122.54 (39.78) | 123.64 (45.80) | 0.764 | 0.634 |
| IL-21 (pg/ml) | 135.78 (67.89) | 135.09 (64.56) | 134.22 (67.78) | 0.664 | 0.632 |
| IL-15 (ng/ml) | 145.89 (13.67) | 147.99 (14.67) | 146.89 (23.78) | 0.543 | 0.562 |
| IL-6 (pg/ml) | 0.87 (2.2) | 0.84 (0.97) | 0.85 (0.34) | 0.986 | 0.632 |
| IL-7 (ng/ml) | 13.89 (14.67) | 13.80 (15.78) | 14.02 (14.75) | 0.895 | 0.763 |
| GM-CSF (ng/ml) | 90.78 (56.78) | 90.88 (55.86) | 90.45 (56.87) | 0.654 | 0.825 |
| IL-2 (ng/ml) | 0.98 (1.23) | 0.99 (1.20) | 1.02 (1.13) | 0.548 | 0.982 |
| IL-17α (ng/ml) | 13.78 (22.78) | 13.89 (23.54) | 12.45 (21.43) | 0.912 | 0.943 |
| MCP (ng/ml) | 39.05 (22.67) | 38.67 (22.64) | 37.85 (23.67) | 0.904 | 0.864 |
| IL-32 (ng/ml) | 109.45 (56.78) | 107.34 (54.67) | 106.67 (55.78) | 0.783 | 0.867 |
| RANTES (ng/ml) | 67.78 (34.09) | 69.74 (33.12) | 69.89 (34.23) | 0.832 | 0.653 |
| Apolipoprotein A1 (ng/ml) | 0.78 (1.32) | 0.88 (0.78) | 0.89 (0.78) | 0.604 | 0.534 |
| Apolipoprotein D (ng/ml) | 0.13 (0.09) | 0.14 (0.12) | 0.13 (0.13) | 0.703 | 0.563 |
| Apolipoprotein E (ng/ml) | 0.08 (0.02) | 0.07 (0.03) | 0.07 (0.04) | 0.956 | 0.673 |
| Ischemia-modified albumin (IMA) (ng/ml) | 0.23 (0.98) | 0.22 (0.67) | 0.23 (0.69) | 0.924 | 0.987 |
| Fibronectin(ng/ml) | 1.45 (1.09) | 1.34 (1.03) | 1.33 (1.04) | 0.743 | 0.763 |
| Aspartate aminotransferase (ng/ml) | 2.45 (1.02) | 2.34 (1.04) | 2.35 (1.06) | 0.605 | 0.235 |
| Lactate dehydrogenase (ng/ml) | 16.78 (10.56) | 16.89 (12.63) | 16.78 (12.89) | 0.894 | 0.673 |
| Tissue factor | 13.56 | 14.09 | 14.15 | 0.231 | 0.983 |
| activity (ng/ml) | (12.34) | (13.56) | (14.09) | | |
| MCP-1 (ng/ml) | 0.34 (0.67) | 0.32 (0.75) | 0.33 (0.74) | 0.342 | 0.893 |
| sVCAM-1 (pg/ml) | 1.56 (0.97) | 1.58 (1.03) | 1.57 (1.12) | 0.453 | 0.673 |
| sCD-40 (ng/ml) | 3.67 (1.34) | 3.69 (1.32) | 3.77 (1.34) | 0.367 | 0.678 |

Conclusion: An optimum set of salivary biomarkers comprising salivary IGF-I, IGF-II, Aβ-40, Aβ-42, alpha amylase, IL-1 beta, and TNF-alpha was determined to be the best subset of biomarkers from a larger database of biomarkers. This subset turns out to be the most appropriate for diagnostic and prognostic purposes as well as monitoring and risk detection for AD.

### Study 2: The Effect of Stimulated and Unstimulated Saliva on IGF-I, IGF-II, Aβ-40, Aβ-42, Alpha amylase, IL-1 beta, and TNF-alpha Biomarker Levels.

Unstimulated saliva samples were collected by a drooling technique and compared to stimulated saliva samples collected by the Salivette® polyester roll device [Sarstedt, Germany] after informed consent and ethical permission was obtained from the required ethical committee, from normal healthy patients and AD sufferers. Samples were tested by ELISA for the following biomarkers: IGF-I, IGF-II, Aβ-40, Aβ-42, alpha amylase, IL-1 beta, and TNF-alpha.

Results: Salivary IGF-I, IGF-II, Aβ-40, Aβ-42, alpha amylase, IL-1 beta, and TNF-alpha levels were non-significantly lower in stimulated saliva compared to unstimulated whole saliva, collected by the drool technique, in both groups (Table-2, p = 0.001).

**Table 2 - Levels of Various Biomarkers in Unstimulated and Whole Stimulated Saliva in AD Sufferers and Normal Healthy Individuals**

| **Mean Value (Standard Deviation)** | | | | |
|---|---|---|---|---|
| Salivary Biomarkers Measured | Control Samples (Normal Healthy Patients) | | AD Patients | |
| | Unstimulated Whole Saliva | Stimulated Saliva | Unstimulated Whole Saliva | Stimulated Saliva |
| Aβ-40 (pg/ml) | 34.4 (4.56) | 29.74 (2.43) | 20.64 (14.67) | 18.86 (12.78) |
| TNF-α (pg/ml) | 68.89 (23.78) | 58.78 (34.56) | 239.67 (45.67) | 214.09 (45.53) |
| IL-1-β (pg/ml) | 48.56 (34.75) | 41.34 (32.67) | 164.23 (47.89) | 155.68 (43.26) |
| Aβ-42 (pg/ml) | 4.08 (2.45) | 3.78 (2.34) | 8.78 (3.21) | 8.65 (2.67) |
| IGF-I (ng/ml) | 2.33 (1.23) | 2.02 (1.12) | 1.23 (0.76) | 1.17 (0.65) |
| IGF-II (ng/dL) | 3.45 (2.78) | 3.23 (2.56) | 2.64 (1.32) | 2.03 (1.64) |
| Alpha-amylase (U/ml) | 20.2 (10.3) | 16.4 (9.7) | 40.6 (12.5) | 32.9 (12.9) |

Conclusion: Unstimulated whole saliva is the preferred specimen for analysis of salivary biomarkers for AD; however stimulated saliva may be used as an alternative specimen. In both unstimulated whole saliva and stimulated saliva specimens there are significant differences between each of the salivary biomarkers in AD patients compared to healthy controls. In certain cases the levels of the biomarkers are increased [e.g. TNF-alpha], while in other cases [e.g. IGF-I] the levels are decreased.

### Study 3: Which Salivary Biomarkers from the Optimized Subset of Salivary Biomarkers, i.e. IGF-I, IGF-II, Aβ-40, Aβ-42, alpha amylase, IL-1 beta, and TNF-alpha are Sufficiently Specific for Use in Mild, Moderate and Severe AD and PD Patients?

AD and PD patients selected for this study were the same as those defined in Study 1. Unstimulated whole saliva specimens were collected and analyzed for salivary biomarkers as for Study 1 above.

Results: Salivary Aβ-42, alpha amylase and IL-1 beta levels were significantly higher in AD patients than in PD patients, while levels of these biomarkers in PD patients were higher than normal healthy control specimens. Levels of the biomarkers IGF-I, IGF-II and Aβ-40 were lower in AD patients compared to PD patients, which in turn were lower than levels in normal healthy control specimens. Biomarker levels for Aβ-42, alpha amylase and IL-1 beta increase as the severity of the disease increases from mild to moderate to severe, but in the case of the biomarkers IGF-I, IGF-II and Aβ-40 these levels decrease as the severity of the disease increases from mild to moderate to severe (p = 0.001, see Table-3).

**Table 3 - Salivary Biomarker Levels in Different Types of AD, PD and Normal Healthy Individuals**

| Mean Value (Standard Deviation) | | | | | |
|---|---|---|---|---|---|
| Salivary Biomarkers Measured | Control Samples (Normal Healthy Individuals) | Parkinson's Disease Patients | Alzheimer's Disease Patients | | |
| | | | Mild | Moderate | Severe |
| Aβ-40 (pg/ml) | 34.4 (4.56) | 23.6 (3.78) | 19.17 (6.68) | 10.98 (3.56) | 5.78 (2.78) |
| TNF-α (pg/ml) | 68.89 (23.78) | 79.89 (12.45) | 178.6 (35.23) | 231.45 (37.89) | 345.28 (67.43) |
| IL-1-β (pg/ml) | 48.56 (34.75) | 67.56 (24.56) | 164.2 (47.89) | 196.67 (36.78) | 235.62 (56.26) |
| Aβ-42 (pg/ml) | 4.08 (2.45) | 5.78 (2.34) | 8.21 (2.67) | 9.89 (2.13) | 15.34 (4.34) |
| IGF-I (ng/ml) | 2.33 (1.23) | 1.87 (0.97) | 2.03 (0.89) | 1.56 (0.65) | 0.67 (0.32) |
| IGF-II (ng/dL) | 3.45 (2.78) | 2.45 (1.78) | 1.67 (0.84) | 1.08 (0.78) | 0.56 (0.34) |
| Alpha-amylase (U/ml) | 20.2 (10.3) | 38.78 (10.8) | 56.8 (15.6) | 68.23 (13.45) | 90.12 (34.5) |

Conclusion: Quantitation of levels of salivary IGF-I, IGF-II, Aβ-40, Aβ-42, alpha amylase, IL-1 beta, and TNF-alpha biomarkers serve as useful biomarkers for diagnosis of different types of AD and PD.

### Study 4: Salivary IGF-I, IGF-II, Aβ-40, Aβ-42, Alpha amylase, IL-1 beta, and TNF-alpha Biomarkers for the Diagnosis of Alzheimer's Disease and Parkinson's Disease

The three patient groups: 100 Alzheimer's disease (AD) patients; 56 elderly non-demented controls without neurological disease or cognitive impairment and 51 Parkinson's disease (PD) patients selected for this study were the same as those defined in Study 1. Unstimulated whole saliva specimens were collected and analyzed for salivary IGF-I, IGF-II, Aβ-40, Aβ-42, alpha amylase, IL-1 beta, and TNF-alpha as for Study 1 above. Statistical comparison of two populations was performed using two-tailed t-test using GraphPad Prism for Windows, v 5.01 (GraphPad Software, San Diego, CA). Receiver operating characteristic curves (ROC) were generated using R (R Foundation for Statistical Computing, Vienna, Austria).

Results and Conclusions: ROC analysis established diagnostic sensitivity and specificity of 82 %, 89% and 95%, in Alzheimer's disease and Parkinson's diseases, respectively (Tables 4 and 5). The Aβ-42, Aβ-40 and IGF-II biomarkers have high diagnostic values for the diagnosis of Alzheimer's disease and Parkinson's disease followed by IGF-I, TNF-alpha, IL-1 beta and alpha amylase in descending order of importance.

**Table 4 - ROC Analysis and Diagnostic performance of IGF-1, IGF-II, Aβ-40, Aβ-42, Alpha Amylase, IL-1 beta, and TNF-alpha Biomarkers in Alzheimer's Disease Patients.**

| Parameters | Aβ-40 | Aβ-42 | IGF-I | IGF-II | Alpha Amylase | IL-1 beta | TNF-alpha |
|---|---|---|---|---|---|---|---|
| ROC AUC | 0.93 | 0.93 | 0.93 | 0.93 | 0.93 | 0.93 | 0.93 |
| Reference Value | 12 pg/ml | 5.2 pg/ml | 1.73 ng/ml | 0.62 ng/dl | 32 U/ml | 150 pg/ml | 172 pg/ml |
| Sensitivity (%) | 82 | 88 | 65 | 70 | 40 | 56 | 64 |
| Specificity (%) | 90 | 90 | 63 | 72 | 42 | 54 | 67 |
| Test Accuracy (%) | 83 | 88 | 60 | 71 | 35 | 52 | 65 |
| Positive Predictive Value (%) | 95 | 95 | 63 | 70 | 40 | 50 | 64 |
| Negative Predictive Value (%) | 64 | 75 | 62 | 70 | 42 | 53 | 67 |

**Table 5 - ROC Analysis and Diagnostic Performance of IGF-I, IGF-II, Aβ-40, Aβ-42, Alpha Amylase, IL-1 beta, and TNF-alpha Biomarkers in Parkinson's Disease Patients.**

| Parameters | Aβ-40 | Aβ-42 | IGF-I | IGF-II | Alpha Amylase | IL-1 beta | TNF-alpha |
|---|---|---|---|---|---|---|---|
| ROC AUC | 0.92 | 0.92 | 0.92 | 0.92 | 0.92 | 0.92 | 0.92 |
| Reference value | 21 pg/ml | 5.0 pg/ml | 0.90 ng/ml | 0.72 ng/dl | 27 U/ml | 62 pg/ml | 73 pg/ml |
| Sensitivity (%) | 80 | 89 | 62 | 72 | 42 | 57 | 65 |
| Specificity (%) | 95 | 95 | 62 | 73 | 40 | 55 | 67 |
| Test Accuracy (%) | 81 | 84 | 61 | 70 | 39 | 53 | 68 |
| Positive Predictive Value (%) | 95 | 95 | 65 | 71 | 38 | 52 | 66 |
| Negative Predictive Value (%) | 70 | 78 | 64 | 73 | 43 | 51 | 66 |

### Study 5: Combination Biomarker Panel of Salivary IGF-I, IGF-II_{.} Aβ-40, Aβ-42, Alpha Amylase, IL-1 beta, and TNF-alpha for the Diagnosis of Alzheimer's Disease and Parkinson's Disease.

The three patient groups in this study, i.e. 100 Alzheimer's disease (AD) patients; 56 elderly non-demented controls without neurological disease or cognitive impairment and 51 Parkinson's disease (PD) patients were the same as those selected in Study 1. Unstimulated whole saliva specimens were collected and analyzed for salivary IGF-I, IGF-II, Aβ-40, Aβ-42, alpha amylase, IL-1 beta, and TNF-alpha as in Study 1 above. Statistical comparison of the two populations [AD and PD] by combination of salivary biomarkers was performed using the two-tailed t-test using GraphPad Prism for Windows, v 5.01 (GraphPad Software, San Diego, CA). Receiver operating characteristic curves (ROC) were generated using R (R Foundation for Statistical Computing, Vienna, Austria). Reference levels used are those described in Study 4 and Tables 4 and 5.

Results and conclusions: ROC analysis established diagnostic sensitivity and specificity for Alzheimer's disease and Parkinson's disease as shown in Tables 6 and 7. The combination models of Aβ-42, Aβ-40, and IGF-II have high diagnostic values for diagnosis of Alzheimer's disease and Parkinson's disease as compared to other combination models.

**Table 6 - ROC Analysis and Diagnostic Performance for Various Biomarker Combinations (IGF-I, IGF-II, Aβ-40, Aβ-42, Alpha Amylase, IL-1 beta, and TNF-alpha) in Alzheimer's Disease Patients.**

| Parameters | Aβ-40 | Aβ-42+ Aβ-40 | IGF-II+ Aβ-42+ Aβ-40 | IGF-I+ IGF-II+ Aβ-42+ Aβ-40 | TNF-alpha+ IGF-I+ IGF-II+ Aβ-42+ Aβ-40 | IL-1 beta+ TNF-alpha+ IGF-I+ IGF-II+ Aβ-42+ Aβ-40 | alpha amylase+ IL-1 beta+ TNF-alpha+ IGF-I+ IGF-II+ Aβ-42+ Aβ-40 |
|---|---|---|---|---|---|---|---|
| ROC AUC | 0.93 | 0.93 | 0.93 | 0.93 | 0.93 | 0.93 | 0.93 |
| Sensitivity (%) | 82 | 89 | 92 | 93 | 94 | 95 | 95 |
| Specificity (%) | 90 | 92 | 94 | 95 | 96 | 96 | 96.2 |
| Test Accuracy (%) | 83 | 93 | 94 | 95 | 95 | 95.6 | 95.7 |
| Positive Predictive Value (%) | 95 | 94 | 95 | 95 | 95 | 96.2 | 96.2 |
| Negative Predictive Value (%) | 64 | 80 | 86 | 90 | 92 | 93 | 93 |

**Table 7 - ROC Analysis and Diagnostic Performance for Biomarker Combinations (IGF-I, IGF-II, Aβ-40, Aβ-42, Alpha Amylase, IL-1 beta, and TNF-alpha) in Parkinson's Disease Patients.**

| Parameters | Aβ-40 | Aβ-42+ Aβ-40 | IGF-II+ Aβ-42+ Aβ-40 | IGF-I+ IGF-II+ Aβ-42+ Aβ**-**40 | TNF-alpha+ IGF-I + IGF-II+ Aβ-42+ Aβ-40 | IL-1 beta+ TNF-alpha+ IGF-I+ IGF-II+ Aβ-42+ Aβ-40 | alpha amylase+ IL-1 beta+ TNF-alpha+ IGF-I+ IGF-II+ Aβ-42+ Aβ-40 |
|---|---|---|---|---|---|---|---|
| ROC AUC | 0.92 | 0.92 | 0.92 | 0.92 | 0.92 | 0.92 | 0.92 |
| Sensitivity (%) | 80 | 82 | 89 | 90 | 92 | 92 | 92 |
| Specificity (%) | 95 | 96 | 96.4 | 96.5 | 96.5 | 96.5 | 96.5 |
| Test Accuracy (%) | 81 | 86 | 87 | 89 | 90 | 90.2 | 92 |
| Positive Predictive Value (%) | 95 | 95 | 95.5 | 95.6 | 95.7 | 96 | 97 |
| Negative Predictive Value (%) | 70 | 80 | 84 | 86 | 91 | 92 | 92 |

### Study 6: AD Diagnostic Biomarkers

In a further experiment, we compared different salivary proteins in AD patients using saliva specimens collected from patients with Alzheimer's disease (with a mean age of 74) in comparison to control saliva specimens collected from control subjects (with a similar mean age, 75). Alzheimer's disease subjects were clinically diagnosed with AD by a neurologist and had Mini Mental State Exam (MMSE) scores ranging from 14-26. Salivary samples were assayed using a sandwich-format ELISA (as described in Study-1).

A comparison of the relative biomarker levels in salivary specimens was made comparing control and AD groups. The results reveal the following discriminatory biomarkers: IGF-I, IGF-II, Aβ-40, Aβ-42, alpha amylase, IL-1 beta, and TNF- alpha. As described elsewhere, in certain embodiments of the invention, the set of AD diagnostic biomarkers could comprise a larger set made up of the following biomarkers, or subsets therefrom: cTnI, myoglobin, MMP-9, MMP-8, MMP-2, sICAM-1, myeloperoxidase [MPO], IL-4, and / or IL-5; B-type natiuretic peptide [BNP], IL-1α, IL-11, IL-10, TNF-α, IFN-γ, VEGF, insulin, GLP-1 (active), GLP-1 (total), TREM1, Leukotriene E4, Akt1, Aβ-40, Aβ-42, Fas ligand, PSA, G-CSF, MIP-1α, IL-22, IL-8, IL-21, IL-15, IL-6, IL-7, GM-CSF, IL-2, IL-12, IL-17α, IL-1β, MCP, IL-32 or RANTES, apolipoproteins A1, D and E, ischemia-modified albumin (IMA), fibronectin, s. alpha-amylase, aspartate aminotransferase, lactate dehydrogenase, tissue factor activity, MCP-1, sVCAM-1, sCD-40, insulin-like growth factor I (IGF-I) and IGF-II.

An unsupervised clustering (that is, the clustering algorithm that is blind to which cases are AD and which are normal) of the 40 discriminatory markers results in the clustering of the samples into 2 groups or clusters, a cluster of control samples, and a cluster of AD samples. Sensitivity in this instance was calculated as the number of correctly classified AD samples in the AD cluster divided by the total number of AD samples, which, in this particular example, is 29/32 or 90.6%.

A comparison was made between biomarker levels in the control and AD groups, revealing eight (8) biomarkers (shown in Table 8) that are differentially regulated between the two groups. Statistical analysis was performed to find the probability that the finding of differential levels was in error (the "q" value) for any one biomarker. A fold difference can be determined by measuring the absolute concentration of a biomarker and comparing that to the absolute value of a reference. Alternately, a fold difference can be measured as the relative difference between a reference value and a sample value, where neither value is a measure of absolute concentration, and / or where both values are measured simultaneously. A fold difference can be a value in the range of 10% to 90%. An ELISA test may be used to measure the absolute content or concentration of a protein from which a fold change is determined in comparison to the absolute concentration of the same biomarker in the reference sample.

Biomarkers with differential levels and associated q values (shown as percentage values) are shown in Table 8 (fold change indicates the fold change between levels in control vs. AD samples). Sensitivity was calculated as the number of AD samples in the AD cluster divided by the total number of AD samples, which works out to be 29/32 or 90.6%. Specificity was calculated as the total correctly predicted AD number divided by the total predicted number of AD patients, which in this case is 29/34 = 85%.

**Table 8: Fold Changes for Salivary Biomarkers in AD.**

| Salivary Biomarker | Fold Change | q-Value (%) |
|---|---|---|
| Aβ-40 (pg/ml) | 0.786 | 1.656 |
| TNF-α (pg/ml) | 0.778 | 1.656 |
| IL-1-β (pg/ml) | 0.784 | 1.656 |
| Aβ-42 (pg/ml) | 0.867 | 1.656 |
| IGF-I (ng/ml) | 0.786 | 1.656 |
| IGF-II (ng/dL) | 0.734 | 1.656 |
| Alpha Amylase (U/ml) | 0.678 | 1.656 |

### Study 7: Decision Trees from the Foregoing AD Diagnostic Biomarker Data

Upon further analysis of the data from Study 4, two different decision trees were formulated for the diagnosis of AD, using AD diagnostic biomarkers. The first decision tree utilizes IGF-I, IGF-II, Aβ-40, Aβ-42, alpha amylase, IL-1 beta, and TNF- alpha levels and the second decision tree utilizes cTnI, myoglobin, MMP-9, MMP-8, MMP-2, sICAM-1 and myeloperoxidase [MPO]. Specificity was calculated from the testing scores as total correctly predicted cases of AD / total number of AD cases (in this case 29/33 cases were correctly identified resulting in a calculated specificity 29/33 = 0.878). Specificity when applying the first decision tree was 98.2% while using the second decision tree resulted in a specificity of only 45.8%.

### Study 8: To Check the Sensitivity and Specificity of Salivary Biomarkers for the Diagnosis of AD.

A total of 100 patients at different stages of AD were selected from an outpatient department of a chosen hospital and each was enrolled in our study following receipt of ethical permission. The diagnosis of patients was made based upon standardized techniques as per the guidelines previously described (see Study 1). Salivary samples were taken and sent for analysis without knowledge of the diagnosis of patients performed by alternate methods. The following salivary biomarkers: IGF-I, IGF-II, Aβ-40, Aβ-42, alpha amylase, IL-1beta, and TNF-alpha levels were analyzed and subsequently patients were categorized according to the criteria described in Study 3 in order to calculate the sensitivities and specificities for each of these specific biomarkers relevant to the diagnosis of AD. Sensitivity was calculated as the number of AD samples in the AD cluster divided by the total number of AD samples, which works out to be 29/32 or 90.6%. Specificity was calculated as the total correctly predicted AD number divided by the total predicted number of AD patients, which in this case is 29/34 = 85%.

Results: The sensitivities calculated for the various biomarkers (using a combination of between two and eight biomarkers) were high, ranging from 87% to 98.9%. The range of specificities was also high (from 87.5%-96.5%), therefore these biomarkers are highly applicable to the diagnosis of the different stages of AD.

### Study 9: Salivary Aβ-42, Aβ-40 and Ratio of Aβ-40 / Aβ-42 as Potential Biomarkers for the Diagnosis of Alzheimer's Disease and Parkinson's Disease.

Three patient groups: 100 Alzheimer's disease (AD) patients; 56 elderly non-demented controls without neurological disease or cognitive impairment and 51 Parkinson's disease (PD) patients selected for this study were the same as those defined in Study 1 above. Unstimulated whole saliva specimens were collected and analyzed for salivary Aβ-42 and Aβ-40 as in Study 1 above. Statistical comparison of the two diseased populations was performed using two-tailed t-test using GraphPad Prism for Windows, v 5.01 (GraphPad Software, San Diego, CA). Receiver operating characteristic curves (ROC) were generated using R (R Foundation for Statistical Computing, Vienna, Austria).

Results: ROC analysis demonstrated diagnostic sensitivity and specificity of 95% and 90%, respectively (Table 9) in the case of Alzheimer's disease. Positive and negative predictive values were estimated to be near 95% and 90%, respectively, whereas the biomarkers Aβ-40 and Aβ-42 alone had negative predictive values that were equal to or less than 75% in the case of Alzheimer's disease. ROC analysis established diagnostic sensitivity and specificity of 95% and 90%, respectively (Table 10) in the case of Alzheimer's disease. Positive and negative predictive values were estimated at approximately 95% and 95%, respectively, whereas Aβ-40 and Aβ-42 biomarkers alone had negative predictive values that were equal to or less than 70% in the case of Parkinson's disease.

**Table 9 - ROC Analysis and Diagnostic Performance of Aβ3-40, Aβ-42, and a Ratio of Aβ-40 / Aβ-42 Biomarkers in Alzheimer's Disease**

| Parameters | Aβ-40 | Aβ-42 | Aβ-40 / Aβ-42 |
|---|---|---|---|
| ROC AUC | 0.93 | 0.93 | 0.98 |
| Reference Value | 21 pg/ml | 9 pg/ml | 3 |
| Sensitivity (%) | 82 | 88 | 96 |
| Specificity (%) | 90 | 90 | 90 |
| Test Accuracy (%) | 83 | 88 | 94 |
| Positive Predictive Value (%) | 95 | 95 | 96 |
| Negative Predictive Value (%) | 64 | 75 | 90 |

**Table 10 - ROC Analysis and Diagnostic Performance of Aβ-40, Aβ-42, and Ratio of Aβ-40 / Aβ-42 Biomarkers in Parkinson's Disease.**

| Parameters | Aβ-40 | Aβ-42 | Aβ-40 / Aβ-42 |
|---|---|---|---|
| ROC AUC | 0.92 | 0.92 | 0.98 |
| Reference Value | 25 pg/ml | 50 pg/ml | 0.5 |
| Sensitivity (%) | 80 | 89 | 95 |
| Specificity (%) | 95 | 95 | 95 |
| Test Accuracy (%) | 81 | 84 | 93 |
| Positive Predictive Value (%) | 95 | 95 | 95 |
| Negative Predictive Value (%) | 70 | 78 | 90 |

Conclusions: Salivary Aβ-42, Aβ-40 and the ratio of Aβ-40 / Aβ-42 are early detection biomarkers for Alzheimer's disease and Parkinson's disease.

### Study 10: Ratios of Aβ-42 / Aβ-40 with Increased Imminent Risk for Mild Cognitive Impairment and Alzheimer's Disease.

Subjects in this study were 100 cognitively normal older adults forming a complete subset of those entering three clinics. These "patients" were registered at the three clinics as normal controls between 2006 and 2011 and had at least 3 stored saliva specimens. For the purpose of this and all other studies discussed herein, cognitively normal adults are defined as community-dwelling, independently functioning individuals who were examined by a medical physician from one of the three clinics and met the following selection criteria: (1) No complaints of memory difficulties during the history taking and medical examination; (2) No active neurologic or psychiatric conditions; (3) No use of psychoactive or psychiatric medications in sufficient quantities to affect cognition; (4) Documented notation confirming that the person's memory was normal.

Individuals with a history of medical conditions or disorders that could affect cognition (e.g. head injuries) were included only if the condition was no longer active and there was no evidence of persistent or residual cognitive impairment. Individuals with current, chronic medical conditions were only included if their medical doctor judged the existing condition to be under control and not affecting cognition.

All subjects underwent baseline neurologic and neuropsychological evaluations such as the Clinical Dementia Rating (Morris JC, "The Clinical Dementia Rating (CDR): Current Version and Scoring Rules," Neurology (1993) 43: 2412-2414); Cummings JL, Mega M, Gray K, Rosenberg-Thompson S, Carusi D, Gornbein J. "The Neuropsychiatric Inventory: Comprehensive Assessment of Psychopathology in Dementia," Neurology (1994) 44: 2308-2314; Kokmen E, Smith G, Petersen R, Tangalos E, Ivnik R, "The Short Test of Mental Status: Correlations with Standardized Psychometric Testing," Arch Neurol. (1991); 48: 725-728); Hachinski VC, Lassen NA, Marshall J. "Hachinski Ischemic Index - Multi-Infarct Dementia: a Cause of Mental Deterioration in the Elderly," Lancet (1974); 2 (7874): 207-210 and >Unified Parkinson's Disease Rating Scale," In: Fahn S, Marsden C, Calne D, Golstein M, eds. "Recent Developments in Parkinson's Disease" New York, NY: MacMillan Publishing Co Inc. (1987); Dementia Rating Scale (DRS) Mattis S. "Dementia Rating Scale: Psychological Assessment Resources," (1983): Auditory Verbal Learning Test: Rey A. "L'examen Psychologique dans les cas d'Encephalopathie Traumatique," Arch Psychol. (1941); 28: 286-340, Wechsler Memory Scale-Revised; Wechsler D. Wechsler "Memory Scale-Revised". New York, NY: Psychological Corporation; (1987); and Wechsler Adult Intelligence Scale- Revised; Wechsler D. "Wechsler Adult Intelligence Scale-Revised". New York, NY: Psychological Corporation; (1981). Study subjects were contacted yearly for re-examination. Medication lists and information regarding family history of dementia were updated at each follow-up visit. Also, the Clinical Dementia Rating, Record of Independent Living, Neuropsychiatric Inventory, Hachinski Ischemic Index, Unified Parkinson's Disease Rating Scale, Kokmen Short Test of Mental Status, and neuropsychological test battery were repeated. All baseline and follow-up examinations were reviewed at monthly consensus conferences. At baseline, entry criteria were reviewed and a Clinical Dementia Rating score of 0 was confirmed for all normal individuals enrolled in the three clinics. Data were compared with those of baseline studies to evaluate progression to MCI or dementia. Possible and probable AD was measured by standardized methods (McKhann G, Drachman D, Folstein M, Katzman R, Price D, Stadlan E.: "Clinical Diagnosis of Alzheimer's Disease: Report of the NINCDS-ADRDA Working Group Under the Auspices of the Department of Health and Human Services Task Force on Alzheimer's Disease," Neurology (1984) 34: 939-944). Subjects with abnormal clinical findings on follow-up but who did not meet the established criteria for MCI or dementia were coded as having cognitive impairment of undetermined origin. We have reported that more than 50% of amnestic [i.e. partial or total loss of memory] MCI cases convert to AD within 5 years. So, with a limited number of patients converting to AD, we combined our end point as incident cases of amnestic MCI and AD patients, which were initially identified during this particular study for the first time. The median follow-up time for this study was 3.2 (1.5) years. Twenty two [22] subjects were diagnosed as having MCI / AD during their follow-up. Of the 22 converters, 12 developed MCI, and 10 of these went on to develop AD (6 probable cases and 4 possible cases). To evaluate the relationship of cognitive change to salivary Aβ-42 and Aβ-40 levels, we identified a subgroup of individuals who had 2 Dementia Rating Scale [DRS] evaluations approximately 4 years apart and a saliva sample taken at the time of the first evaluation. Patients also had to be cognitively normal at the first time point. A window of 4 (±1) years was used for the time between DRS evaluations, and a window of ±7 months was used for the time between the initial visit and the date the saliva sample was obtained. Levels of salivary Aβ-42 and Aβ-40 were measured as described in Study-1. The Kaplan-Meier method was used to estimate the distribution of time to development of MCI / AD, with the time of the first collected saliva sample considered as the start of follow-up.

Results: The salivary Aβ-42/ Aβ-40 ratio showed evidence of an association with the conversion to MCI/AD (Table 11). The risk of MCI/AD for patients with an Aβ-42/ Aβ-40 ratio in the lowest quartile was estimated to be 3 times higher than the risk for subjects with a ratio in the highest quartile (P=.01). Subjects whose Aβ-42 / Aβ-40 ratio was in the lowest quartile (Q1, p <0.01) reached a 10% incidence after a period of 5 years, followed by those in Q2 who took approximately 7 years, and those in Q3 and Q4 who took approximately 10 years to reach 10% cumulative incidence.

**Table 11 - Time to MCI or AD and Salivary Aβ Ratio Measurement**

| Variable | Single Variable model | |
|---|---|---|
| | RR (95%CI) | P value |
| Aβ-42/ Aβ-40 | | |
| Less than the Median | 1.68 (1.04-3.67) | 0.04 |
| Quartiles | | |
| Q1 | 3.87 (1.45-6.98) | 0.01 |
| Q2 | 2.97 (1.07-6.23) | |
| Q3 | 2.03 (0.98-5.99) | |
| Q4 | 1.00 | |
| Log Ratio | 1.56 (1.00-2.34) | 0.046 |
| Log Ratio Extremes Removed | 1.54 (0.86-2.56) | 0.10 |

Conclusion: The ratio of salivary levels of Aβ-42 / Aβ-40 may be used for risk detection and diagnosis of AD and MCI.

### Study 11: Brain Autopsy Confirmed AD and Salivary Biomarkers

All subjects in this part of the work were part of a clinical cohort. Each subject underwent a standard evaluation, including medical history, physical / neurological examination and a neuropsychological battery (Stern Y, Andrews H, Pittman J, et al.: "Diagnosis of Dementia in a Heterogeneous Population-Development of a Neuropsychological Paradigm-based Diagnosis of Dementia and Quantified Correction for the Effects of Education," Arch Neurol (1992) 49:453-60). For this study we used data from 10 subjects who died during the study period, had detailed semi-quantitative data from brain autopsy and at least two complete assessments prior to death. Neuropathological evaluation was performed blinded to the clinical data. One half-brain of each patient was assessed grossly while it was dissected in the fresh state to harvest blocks which were deep frozen and banked. The contralateral half of the brain was immersed in 10% buffered formalin phosphate solution for neuropathological evaluation, as described (Vonsattel JP, Aizawa H, Ge P, et al. "An Improved Approach to Prepare Human Brains for Research," J Neuropathol Exp Neurol (1995) 54: 42-56). Unstimulated saliva samples were collected from all subjects at least 2-3 months before the death of the subjects. Salivary IGF-I, IGF-II, Aβ-40, Aβ-42, Alpha amylase, IL-1 beta, and TNF-alpha biomarker levels were measured as described in Study-1.

Results: Salivary IGF-I, IGF-II, Aβ-40, Aβ-42, alpha amylase, IL-1 beta, and TNF-alpha biomarker levels were significantly different in AD (Table 12) compared to normal controls.

**Table 12 - Salivary Biomarkers in Brain Autopsy Confirmed AD.**

| Salivary Biomarkers Measured | AD Patients |
|---|---|
| | Unstimulated Whole Saliva |
| Aβ-40 (pg/ml) | 17.67 (11.70) |
| TNF-α (pg/ml) | 345.12 (49.52) |
| IL-1-β (pg/ml) | 178.64 (41.24) |
| Aβ-42 (pg/ml) | 9.78 (1.78) |
| IGF-I (ng/ml) | 1.08 (0.56) |
| IGF-II (ng/dL) | 1.89 (1.56) |
| Alpha Amylase (U/ml) | 45.5 (12.4) |

Conclusion: Quantitation of levels of salivary IGF-I, IGF-II, Aβ-40, Aβ-42, alpha amylase, IL-1 beta, and TNF-alpha biomarkers serve as useful biomarkers for the diagnosis of different types of AD.

### Study 12: Salivary and CSF biomarkers in maternal history of late-onset Alzheimer's disease individuals

Twenty-five (25) cognitively normal individuals with a maternal history of late-onset Alzheimer's disease individuals were selected as test subjects in this study. The test subjects were subjected to clinical, neuropsychological, MRI exams and a lumbar puncture and saliva biomarkers were measured within 3 a month period.

The neuropsychological exam was conducted using neuropsychological testing battery and Global Deterioration Scale (De Santi S, Pirraglia E, Barr WB, Babb J, Williams S, Rogers K, et al. Robust and conventional neuropsychological norms: Diagnosis and prediction of age-related cognitive decline. Neuropsychology. 2008; 22:469-484; Reisberg B, Ferris SH, de Leon MJ, Crook T. The global deterioration scale for assessment of primary degenerative dementia. Am J Psychiat. 1982; 139:1136-1139).

CSF examination was done in the following manner. The 20 cc of clear CSF were collected using fluoroscopy to guide a 24 gauge beveled LP needle. The CSF Aβ1-40 and Aβ1-42 was measured (Hansson O, Zetterberg H, Buchhave P, Andreasson U, Londos E, Minthon L, et al. Prediction of Alzheimer's disease using the CSF Abeta42/Abeta40 ratio in patients with mild cognitive impairment. Dement Geriatr Cogn Disord. 2007; 23:316-320.). The CSF T-tau measurements were measured according to the methods described in (Hansson O, Zetterberg H, Buchhave P, Londos E, Blennow K, Minthon L. Association between CSF biomarkers and incipient Alzheimer's disease in patients with mild cognitive impairment: a follow-up study. Lancet Neurol. 2006; 5:228-234.). Unstimulated whole saliva specimens were collected and analyzed for salivary biomarkers as for Study 1 above. The correlation between Aβ40, Aβ42, Aβ42/40 in saliva and CSF was determined. Data were analyzed by using SPSS 12.0 (SPSS Inc., Chicago, MI).

**Table 13: Demographical data, CSF and salivary biomarkers in subjects with maternal history of late onset Alzheimer's disease.**

| Mean Value (Standard Deviation) | |
|---|---|
| **Variables** | |
| N | 25 |
| Age (years) | 54(12) |
| Gender | 13 M: 12 F |
| Subjective memory complaints (yes/no) | 20/5 |
| | |

| **Neuropsychological measures** | |
|---|---|
| MMSE | 29.5(1.4) |
| Object naming | 52(12) |
| Paragraph delayed recall | 10(3) |
| Paired associates delayed recall | 7(2) |
| Visual recognition | 18(8) |
| | |

| **Biomarkers** | |
|---|---|
| CSF Aβ₄₀ measures (pg/ml) | 7212(2106) |
| CSF Aβ₄₂ measures (pg/ml) | 876(145) |
| Salivary Aβ₄₀ measures (pg/ml) | 23.24(3.21) |
| Salivary Aβ₄₂ measures (pg/ml) | 9.44(1.02) |
| Salivary TNF-alpha (pg/ml) | 243.78(21.23) |
| Salivary IL-1-β (pg/ml) | 242.32(54.32) |
| Salivary IGF-I (ng/ml) | 0.63(0.31) |
| Salivary IGF-II (ng/dL) | 0.52(0.31) |
| Salivary Alpha-amylase (IU/ml) | 97.21(313) |

Significant correlation between CSF and salivary biomarkers (r2=0.82 to 0.93) was found. The salivary biomarkers levels were found out of the normal healthy range (as shown in Table: 2). So, these biomarkers are useful for detection of risk of AD and detection of a family history of AD.

Conclusions: Salivary IGF-I, IGF-II, Aβ-40, Aβ-42, Alpha Amylase, IL-1 beta, and TNF-alpha serve as useful biomarkers of detection of risk of AD and detection of a family history of AD.

### Field Test Methods

Based on the studies discussed above, accurate field testing methods can be used by practitioners to diagnose AD and PD in the field. The methods all have the common steps of testing a saliva sample for levels of two or more of a group of biomarkers consisting of IGF-I, IGF-II, Aβ-40, Aβ-42, alpha amylase, IL-1 beta, and TNF-alpha, then determining the saliva sample is positive if levels of the group of biomarkers pass two or more criteria in a group of test criteria. For an AD test, the test criteria is if the tested levels of biomarkers are above or below (depending on the biomarker) biomarker reference levels. In some embodiments, the reference levels are those disclosed in Table 4. In other embodiments, the reference levels can be any value in a ± 10% range around the reference levels disclosed in Table 4. For a PD test, the test criteria is when tested levels of the biomarkers fall within a ± 10% range around the average biomarker values for PD disclosed in Table 4. Similarly, for a severe AD test, the test criteria is when tested levels of the biomarkers fall within a ± 10% range around the average biomarker values for severe AD disclosed in Table 4.

### Field Test Kits

Field test kits that carry out the field test methods can be made in many forms. One embodiment of a field test kit has a set of test strips and a reading device. The set of test strips has a type of test strip for each biomarker in a group of biomarkers to be tested, such as the biomarkers in Table 3. Each type of test strip is configured to produce a fluorescence level proportional to a level present on the test strip of one of the group of biomarkers. The reading device is configured to read the fluorescence levels on each of the test strips and configured to indicate a positive result when the fluorescence levels from each of the test strips are fluorescence levels of test strips exposed to a saliva sample with levels of a group of biomarkers in the saliva sample passing one or more criteria in a group of test criteria. The test criteria can be the test criteria disclosed in the field test methods above.

Those skilled in the art will recognize that numerous modifications and changes may be made to the preferred embodiment without departing from the scope of the claimed invention. It will, of course, be understood that modifications of the invention, in its various aspects, will be apparent to those skilled in the art, some being apparent only after study, others being matters of routine mechanical, chemical and electronic design. No single feature, function or property of the preferred embodiment is essential. Other embodiments are possible, their specific designs depending upon the particular application. As such, the scope of the invention should not be limited by the particular embodiments herein described but should be defined only by the appended claims.

## Claims

1. A method to test for Alzheimer's disease, comprising the steps of:
testing a saliva sample for levels of a group of biomarkers consisting of IGF-I, IGF-II, Aβ-40, Aβ-42, alpha amylase, IL-1 beta, and TNF-alpha; and
determining the saliva sample is positive for Alzheimer's disease if the levels of the group of biomarkers pass all criteria in a group of test criteria consisting of:
Aβ-40 level below an Aβ-40 reference level,
TNF-alpha level above a TNF-alpha reference level,
IL-1 beta level above an IL-1 beta reference level,
Aβ-42 level above an Aβ-42 reference level,
IGF-I level below an IGF-I reference,
IGF-II level below an IGF-II reference level, and
Alpha-amylase level above an Alpha-amylase reference level.

2. The method of claim 1, wherein:
the Aβ-40 reference level is between 10.8 and 13.2 pg/ml,
the TNF-alpha reference level is between 154.8 and 189.2 pg/ml,
the IL-1 beta reference level is between 135 and 165 pg/ml,
the Aβ-42 reference level is between 4.68 and 5.72 pg/ml,
the IGF-I reference level is between1.56 and 1.9 ng/ml,
the IGF-II reference level is between0.56 and 0.68 ng/dl, and
the Alpha-amylase reference level is between 28.8 and 35.2 U/ml.

3. A method to test for severe Alzheimer's disease, comprising the steps of:
testing a saliva sample for levels of a group of biomarkers consisting of IGF-I, IGF-II, Aβ-40, Aβ-42, alpha amylase, IL-1 beta, and TNF-alpha; and
determining the saliva sample is positive for severe Alzheimer's disease if the levels of the group of biomarkers pass all criteria in a group of test criteria consisting of:
a Aβ-40 level is between 5.20 and 6.36 pg/ml,
a TNF-alpha level is between 310.8 and 379.8 pg/ml,
a IL-1 beta level is between 212.1 and 259.2 pg/ml,
a Aβ-42 level is between 13.9 and 17.0 pg/ml,
a IGF-I level is between 0.60 and 0.74 ng/ml,
a IGF-II level is between 0.50 and 0.62 ng/dl, and
a Alpha-amylase level is between 81.1 and 99.1 U/ml.

4. A method to test for moderate Alzheimer's disease, comprising the steps of:
testing a saliva sample for levels of a group of biomarkers consisting of IGF-I, IGF-II, Aβ-40, Aβ-42, alpha amylase, IL-1 beta, and TNF-alpha; and
determining the saliva sample is positive for moderate Alzheimer's disease if the levels of the group of biomarkers pass all criteria in a group of test criteria consisting of:
a Aβ-40 level is between 9.88 and 12.08 pg/ml,
a TNF-alpha level is between 208.31 and 254.6 pg/ml,
a IL-1 beta level is between 177 and 216 pg/ml,
a Aβ-42 level is between 8.90 and 10.9 pg/ml,
a IGF-I level is between 1.40 and 1.72 ng/ml,
a IGF-II level is between 0.97 and 1.19 ng/dl, and
a Alpha-amylase level is between 61.4 and 75.0 U/ml.

5. A method to test for Parkinson's disease, comprising the steps of:
testing a saliva sample for levels of a group of biomarkers consisting of IGF-I, IGF-II, Aβ-40, Aβ-42, alpha amylase, IL-1 beta, and TNF-alpha; and
determining the saliva sample is positive for Parkinson's disease if levels of the group of biomarkers pass all criteria in a group of test criteria consisting of:
a Aβ-40 reference level is between 21.2 and 25.9 pg/ml,
a TNF-alpha reference level is between 71.9 and 87.9 pg/ml,
a IL-1 beta reference level is between 60.8 and 74.3 pg/ml,
a Aβ-42 reference level is between 5.2 and 6.4 pg/ml,
a IGF-I reference level is between 1.68 and 2.06 ng/ml,
a IGF-II reference level is between 2.21 and 2.69 ng/dl, and
a Alpha-amylase reference level is between 34.9 and 42.7 U/ml.

## Patentansprüche

1. Verfahren zum Testen auf Alzheimer-Krankheit, umfassend die Schritte:
Testen einer Speichelprobe bezüglich der Level einer Gruppe von Biomarkern, bestehend aus IGF-I, IGF-II, Aβ-40, Aβ-42, Alpha-Amylase, IL-1 beta und TNF-alpha; und
Bestimmen, dass die Speichelprobe bezüglich der Alzheimer-Krankheit positiv ist, wenn die Level der Gruppe von Biomarkern alle Kriterien in einer Gruppe von Testkriterien, bestehend aus:
Aβ-40 Level unterhalb eines Aβ-40 Referenzlevels,
TNF-alpha Level oberhalb eines TNF-alpha Referenzlevels,
IL-1 beta Level oberhalb eines IL-1 beta Referenzlevels,
Aβ-42 Level oberhalb eines Aβ-42 Referenzlevels,
IGF-I Level unterhalb einer IGF-I Referenz,
IGF-II Level unterhalb eines IGF-II Referenzlevels und
Alpha-Amylase Level oberhalb eines Alpha-Amylase Referenzlevels, erfüllen.

2. Verfahren gemäß Anspruch 1, wobei:
der Aβ-40 Referenzlevel zwischen 10,8 und 13,2 pg/ml liegt,
der TNF-alpha Referenzlevel zwischen 154,8 und 189,2 pg/ml liegt,
der IL-1 beta Referenzlevel zwischen 135 und 165 pg/ml liegt,
der Aβ-42 Referenzlevel zwischen 4,68 und 5,72 pg/ml liegt,
der IGF-I Referenzlevel zwischen 1,56 und 1,9 ng/ml liegt,
der IGF-II Referenzlevel zwischen 0,56 und 0,68 ng/dl liegt und
der Alpha-Amylase Referenzlevel zwischen 28,8 und 35,2 U/ml liegt.

3. Verfahren zum Testen auf schwere Alzheimer-Krankheit, umfassend die Schritte:
Testen einer Speichelprobe bezüglich der Level einer Gruppe von Biomarkern, bestehend aus IGF-I, IGF-II, Aβ-40, Aβ-42, Alpha-Amylase, IL-1 beta und TNF-alpha; und
Bestimmen, dass die Speichelprobe bezüglich schwerer Alzheimer-Krankheit positiv ist, wenn die Level der Gruppe von Biomarkern alle Kriterien in einer Gruppe von Testkriterien, bestehend aus:
einem Aβ-40 Level, der zwischen 5,20 und 6,36 pg/ml liegt,
einem TNF-alpha Level, der zwischen 310,8 und 379,8 pg/ml liegt,
einem IL-1 beta Level, der zwischen 212,2 und 259,2 pg/ml liegt,
einem Aβ-42 Level, der zwischen 13,9 und 17,0 pg/ml liegt,
einem IGF-I Level, der zwischen 0,60 und 0,74 ng/ml liegt,
einem IGF-II Level, der zwischen 0,50 und 0,62 ng/dl liegt, und
einem Alpha-Amylase Level, der zwischen 81,1 und 99,1 U/ml liegt, erfüllen.

4. Verfahren zum Testen auf mäßige Alzheimer-Krankheit, umfassend die Schritte:
Testen einer Speichelprobe bezüglich der Level einer Gruppe von Biomarkern, bestehend aus IGF-I, IGF-II, Aβ-40, Aβ-42, Alpha-Amylase, IL-1 beta und TNF-alpha; und
Bestimmen, dass die Speichelprobe bezüglich mäßiger Alzheimer-Krankheit positiv ist, wenn die Level der Gruppe von Biomarkern alle Kriterien in einer Gruppe von Testkriterien, bestehend aus:
einem Aβ-40 Level, der zwischen 9,88 und 12,08 pg/ml liegt,
einem TNF-alpha Level, der zwischen 208,31 und 254,6 pg/ml liegt,
einem IL-1 beta Level, der zwischen 177 und 216 pg/ml liegt,
einem Aβ-42 Level, der zwischen 8,90 und 10,9 pg/ml liegt,
einem IGF-I Level, der zwischen 1,40 und 1,72 ng/ml liegt,
einem IGF-II Level, der zwischen 0,97 und 1,19 ng/dl liegt, und
einem Alpha-Amylase Level, der zwischen 61,4 und 75,0 U/ml liegt, erfüllen.

5. Verfahren zum Testen auf Parkinson-Krankheit, umfassend die Schritte:
Testen einer Speichelprobe bezüglich der Level einer Gruppe von Biomarkern, bestehend aus IGF-I, IGF-II, Aβ-40, Aβ-42, Alpha-Amylase, IL-1 beta und TNF-alpha; und
Bestimmen, dass die Speichelprobe bezüglich Parkinson-Krankheit positiv ist, wenn die Level der Gruppe von Biomarkern alle Kriterien in einer Gruppe von Testkriterien, bestehend aus:
einem Aβ-40 Referenzlevel, der zwischen 21,2 und 25,9 pg/ml liegt,
einem TNF-alpha Referenzlevel, der zwischen 71,9 und 87,9 pg/ml liegt,
einem IL-1 beta Referenzlevel, der zwischen 60,8 und 74,3 pg/ml liegt,
einem Aβ-42 Referenzlevel, der zwischen 5,2 und 6,4 pg/ml liegt,
einem IGF-I Referenzlevel, der zwischen 1,68 und 2,06 ng/ml liegt,
einem IGF-II Referenzlevel, der zwischen 2,21 und 2,69 ng/dl liegt, und
einem Alpha-Amylase Referenzlevel, der zwischen 34,9 und 42,7 U/ml liegt, erfüllen.

## Revendications

1. Méthode de diagnostic de la maladie d'Alzheimer, comprenant les étapes de :
tester un échantillon de salive pour les taux d'un groupe de biomarqueurs consistant en IGF-I, IGF-II, Aβ-40, Aβ-42, alpha amylase, IL-1 beta, et TNF-alpha ; et
déterminer si l'échantillon de salive est positif pour la maladie d'Alzheimer si les taux du groupe de biomarqueurs répondent à tous les critères dans un groupe de critères de test consistant en :
taux en Aβ-40 inférieur à un taux de référence en Aβ-40,
taux en TNF-alpha supérieur à un taux de référence en TNF-alpha,
taux en IL-1 beta supérieur à un taux de référence en IL-1 beta,
taux en Aβ-42 supérieur à un taux de référence en Aβ-42,
taux en IGF-I inférieur à une référence en IGF-I,
taux en IGF-II inférieur à un taux de référence en IGF-II, et
taux en alpha-amylase supérieur à un taux de référence en alpha-amylase.

2. Méthode selon la revendication une, dans laquelle :
le taux de référence en Aβ-40 est entre 10.8 et 13.2 pg/ml,
le taux de référence en TNF-alpha est entre 154.8 et 189.2 pg/ml,
le taux de référence en IL-1 beta est entre 135 et 165 pg/ml,
le taux de référence en Aβ-42 est entre 4.68 et 5.72 pg/ml,
le taux de référence en IGF-I est entre 1.56 et 1.9 ng/ml,
le taux de référence en IGF-II est entre 0.56 et 0.68 ng/dl, et
le taux de référence en alpha-amylase est entre 28.8 et 35.2 U/ml.

3. Méthode de diagnostic pour la maladie d'Alzheimer aigüe, comprenant les étapes de :
tester un échantillon de salive pour les taux d'un groupe de biomarqueurs consistant en IGF-I, IGF-II, Aβ-40, Aβ-42, alpha amylase, IL-1 beta, et TNF-alpha ; et
déterminer si l'échantillon de salive est positif pour la maladie d'Alzheimer aiguë si les taux du groupe de biomarqueurs répondent à tous les critères dans un groupe de critères de test consistant en :
un taux en Aβ-40 compris entre 5.20 et 6.36 pg/ml,
un taux en TNF-alpha compris entre 310.8 et 379.8 pg/ml,
un taux en IL-1 beta compris entre 212.1 et 259.2 pg/ml,
un taux en Aβ-42 compris entre 13.9 et 17.0 pg/ml,
un taux en IGF-I compris entre 0.60 et 0.74 ng/ml,
un taux en IGF-II compris entre 0.50 et 0.62 ng/dl, et
un taux en alpha-amylase compris entre 81.1 et 99.1 U/ml.

4. Méthode de diagnostic de la maladie d'Alzheimer modérée, comprenant les étapes de :
tester un échantillon de salive pour les taux d'un groupe de biomarqueurs consistant en IGF-I, IGF-II, Aβ-40, Aβ-42, alpha amylase, IL-1 beta, et TNF-alpha ; et
déterminer si l'échantillon de salive est positif pour la maladie d'Alzheimer modérée si les taux du groupe de biomarqueurs répondent à tous les critères dans un groupe de critères de test consistant en :
un taux en Aβ-40 compris entre 9.88 et 12.08 pg/ml,
un taux en TNF-alpha compris entre 208.31 et 254.6 pg/ml,
un taux en IL-1 beta compris entre 177 et 216 pg/ml,
un taux en Aβ-42 compris entre 8.90 et 10.9 pg/ml,
un taux en IGF-I compris entre 1.40 et 1.72 ng/ml,
un taux en IGF-II compris entre 0.97 et 1.19 ng/dl, et
un taux en alpha-amylase compris entre 61.4 et 75.0 U/ml.

5. Méthode de diagnostic de la maladie de Parkinson, comprenant les étapes de :
tester un échantillon de salive pour les taux d'un groupe de biomarqueurs consistant en IGF-I, IGF-II, Aβ-40, Aβ-42, alpha amylase, IL-1 beta, et TNF-alpha ; et
déterminer si l'échantillon de salive est positif pour la maladie de Parkinson si les taux du groupe de biomarqueurs répondent à tous les critères dans un groupe de critères de test consistant en :
un taux de référence en Aβ-40 compris entre 21.2 et 25.9 pg/ml,
un taux de référence en TNF-alpha compris entre 71.9 et 87.9 pg/ml,
un taux de référence en IL-1 beta compris entre 60.8 et 74.3 pg/ml,
un taux de référence en Aβ-42 compris entre 5.2 et 6.4 pg/ml,
un taux de référence en IGF-I compris entre 1.68 et 2.06 ng/ml,
un taux de référence en IGF-II compris entre 2.21 et 2.69 ng/dl, et
un taux de référence en alpha-amylase compris entre 34.9 et 42.7 U/ml.
